# EUROPEAN PATENT APPLICATION

(11) **EP 4 721 858 A1**
(43) Date of publication of application: **08.04.2026**
(21) Application number: 24204147.3
(22) Date of filing: 02.10.2024
(51) Int. Cl.: B01L 3/00, B01L 7/00, C12Q 1/686

(54) **NUCLEIC ACID CONTAMINATION-FREE CARTRIDGE FOR STERILITY TESTING**

(71) Applicant: Sartorius Lab Instruments GmbH & Co. KG, 37079 Göttingen (DE)
(72) Inventor: Hertel, Robert, 37079 Göttingen (DE); Dormeyer, Miriam, 37079 Göttingen (DE); Schmitz, Natalie, 37079 Göttingen (DE); Binninger, Steven, 37079 Göttingen (DE); Müller-Scholz, Alexandra, 37079 Göttingen (DE); Pflanz, Karl, 37079 Göttingen (DE)
(74) Representative: Eisenführ Speiser

(57) **Abstract**

The invention relates to a cartridge for performing a nucleic acid amplification test of sterility on a sample, wherein the cartridge and all components are nucleic acid contamination-free. The cartridge is suitable for processing biological samples, whereas the nucleic acid sterility test detects all clinically relevant bacterial and/or fungal species. Further, the invention relates to a use of said cartridge for performing a nucleic acid amplification test and for detecting at least one fungal and/or at least one bacterial contamination. The invention also relates to a method of producing said cartridge in a nucleic-acid free environment and optionally with DNA-depleted components. Further, the invention relates to a kit or a kit of parts comprising at least one nucleic acid contamination-free nucleic acid amplification reagent. The invention also relates to a device comprising said cartridge.

## Description

### Technical Field

The invention relates to a cartridge for performing a nucleic acid amplification test of sterility on a sample, wherein the cartridge and all components are nucleic acid contamination-free. The cartridge is suitable for processing biological samples, whereas the nucleic acid sterility test detects all clinically relevant bacterial and/or fungal species. Further, the invention relates to a use of said cartridge for performing a nucleic acid amplification test and for detecting at least one fungal and/or at least one bacterial contamination. The invention also relates to a method of producing said cartridge in a nucleic-acid free environment and optionally with DNA-depleted components. Further, the invention relates to a kit or a kit of parts comprising at least one nucleic acid contamination-free nucleic acid amplification reagent. The invention also relates to a device comprising said cartridge.

### Background of the Invention

In modern healthcare, biomedical research and the manufacture of (bio)pharmaceuticals, ensuring the sterility of biological samples, is absolutely essential. Meticulous sterility testing is imperative to safeguard patient safety and guarantee the efficacy of medical treatments. Ensuring sterility in pharmaceutical and medical products or biopharmaceuticals like ATMPs (Advanced Therapy Medicinal Products) is fundamental to prevent infections that could lead to adverse immune reactions, rejection and/or severe complications such as sepsis. Notably, rapid sterility testing is crucial for samples with short shelf lives, such as autologous cell therapy products and radiopharmaceuticals. These products are highly sensitive and often have limited time windows for safe and effective use. Rapid and reliable sterility testing methods are essential to ensure their safety and efficacy before they reach patients, and any delay in testing could render these products unusable, wasting valuable resources and potentially compromising patient outcomes. In the fields of stem cell therapies, tissue-engineered products, and regenerative medicine, the use of sterile products is crucial to prevent the onset of infections, especially in immunocompromised patients. Additionally, blood and marrow transplants, which are vital for treating serious cancers and blood disorders, depend on stringent sterility controls to shield patients from potentially fatal infections. Ensuring sterility in these products is not just a regulatory requirement but a foundational aspect of patient care in these critical treatments.

With the increasing complexity of medical procedures and the rise in the number of immunocompromised patients, ensuring the sterility of biological samples is pivotal to prevent infections by relying on accurate diagnostic results. (Azoulay et al., Focus on immunocompromised patients, Intensive Care Med (2016), 42, 463-465, Fishman, Jay A., Infections in Immunocompromised Hosts and Organ Transplant Recipients: Essentials, Liver Transplantation (2011), 17, 34-37).

The current gold standard for sterility testing continues to be growth-based methods, which involve culturing samples to detect the presence of viable microorganisms. Nucleic acid amplification is not yet widely adopted for sterility testing, as traditional bench-top PCR testing kits are fraught with challenges, primarily due to the high risk of nucleic acid contamination, mainly introduced during manual pipetting procedures.

In a typical laboratory, nucleic acids like DNA or RNA can easily contaminate samples, leading to false-positive results, especially in sterility testing where contaminant DNA can be mistaken for microbial contamination. Manual handling introduces risks such as pipetting errors and cross-contamination, compromising test integrity. The high sensitivity of nucleic acid amplification methods makes them prone to even trace environmental contamination, requiring stringent aseptic techniques, clean rooms, and rigorous controls, all of which are challenging and costly to maintain. Ensuring sterility demands significant resources and vigilance, as even minor lapses can have serious implications for patient safety and treatment outcomes.

To effectively reduce the reliance on highly skilled personnel whilst maintaining highest standards of sterility, implementing an automated solution is crucial. This can be realized through advanced pipetting robots or by fully integrating the process within a cartridge system. While several cartridge-based automation solutions are available for other applications, such as the BIOFIRE Mycoplasma System by BioMérieux, or in the *in vitro* diagnostics (IVD) market, GeneXpert^{®} Systems by Cepheid, and the Vivalytic System by BHCS, and many more - none currently address the specific needs of sterility testing or are suitable for it.

A fully-integrating testing procedure in a cartridge refers to a self-contained, automated system where all steps of a diagnostic and/or analytical process are performed within a single, enclosed cartridge or device. This includes sample preparation, reagent mixing, reaction processes (such as amplification or detection), and result analysis. Such a cartridge may preferably be designed to integrate all necessary components and workflows to minimize the need for external intervention or handling.

Additionally, a significant challenge is the interference of ubiquitous bacterial or fungal nucleic acids such as DNA or RNA, which even affects negative controls. This contamination makes it particularly difficult to detect very low microbial counts (<100 CFU), as the nucleic acid from such contamination cannot be easily distinguished from the ever-present bacterial or fungal nucleic acids in the environment. This limitation underscores the urgent need for more refined and specialized sterility testing solutions.

Furthermore, these existing solutions fall short in meeting critical requirements for high cell density (at least 10 x 10⁶ or at least an equivalent quantity of non-targeted nucleic acids) and sufficient sample volume (up to 5 mL). This is particularly vital for advanced therapeutics, including ATMPs and cell-based medical products: insufficient sample volumes may fail to contain detectable levels of microbial nucleic acids, particularly in scenarios characterized by low-level contamination. Conversely, the utilization of larger sample volumes necessitates more extensive processing, potentially complicating the testing procedure. Furthermore, achieving homogeneity within the sample is imperative, especially given the possibility of uneven microbial contamination distribution. Inadequate homogenization can induce sampling bias, possibly resulting in false negatives or the underestimation of contamination levels, which could have dire implications for patients receiving these biological materials. Consequently, manual sample preparation by highly qualified personnel is still necessary, despite the use of cartridge-based solutions.

Biological sample specific challenges significantly compound the difficulties associated with nucleic acid-based sterility testing. The complex matrices inherent to some samples can inhibit nucleic acid amplification processes, thus increasing the likelihood of false negatives or diminishing the overall sensitivity of the assay. This issue is often aggravated by the requirement for extensive lysis to liberate microbial DNA or RNA from those samples. Incomplete lysis may lead to suboptimal extraction of microbial nucleic acids, thereby reducing the assay's sensitivity. Additionally, the abundant presence of host DNA or RNA in biological samples can obscure the microbial nucleic acids that are the target of detection, further complicating the accurate assessment of sterility.

Interpreting the results of nucleic acid sterility testing also poses significant challenges. Establishing thresholds for detection that differentiate between true contamination and background noise is critical but can be difficult, particularly when dealing with low-level contamination. Furthermore, not all detected nucleic acids are necessarily indicative of a sterility breach. For instance, the presence of DNA from dead microorganisms may not pose a sterility threat, yet it can still lead to positive results, complicating the interpretation and potentially leading to unnecessary concerns or actions.

It was shown before that point-of-care molecular diagnostics can achieve a semi-automated nucleic acid analysis in a closed system, through technologies like microfluidics. With fluid networks incorporating micro-valves, micro-channels, micro-reactors, and micro-actuators, nucleic acid testing can be conducted on a single microfluidic chip, typically supported by a portable instrument (Donglin et al., A microfluidic system for rapid nucleic acid analysis based on real-time convective PCR at point-of-care testing, Microfluidics and Nanofluidics (2022), 26, 69, 1-9). However, this technique is not suitable for broad nucleic acid sterility testing of complex biological samples, as its design and application are more focused on rapid, point-specific diagnostics rather than comprehensive sterility assessments. Further, the use of capillary tubes severely limits the sample type and volume, restricting the application of these PCR systems to specific scenarios. The small inner diameter (1.6 mm) and limited reactor length (19 mm) of these tubes and the micro-sized channelling constrain the amount of sample that can be processed, making it challenging to work with complex or viscous samples at a larger volume.

The problems of only a limited sample volume are also apparent in EP 1 740 721 B1, which a PCR process for DNA amplification within a closed, single-use cartridge, containing micro-channels or micro-cavities, where all reagents are stored at in a dried form. For nucleic acid analysis an additional sample preparation step is required where the cells must first be lysed to release the DNA. Thus, this extra step adds time and effort to the workflow, complicating the overall analysis and increasing the risk of contamination, which makes the disclosed system unsuitable for reliable sterility testing.

Given the numerous challenges associated with traditional bench-top PCR testing kits, there is a clear need for advanced solutions that can mitigate the risks of nucleic acid contamination and enhance the reliability of sterility testing. The susceptibility of PCR to even trace amounts of environmental DNA or RNA demands more robust and automated systems that minimize manual handling and the associated risks of contamination. Additionally, the complexity of biological samples and the high sensitivity of nucleic acid amplification methods call for improved sample processing techniques that ensure thorough homogenization, effective lysis, and accurate detection of microbial nucleic acids without interference from host DNA. To address the issues of cross-contamination and sample volume inconsistencies, the development of closed-system testing devices that streamline and standardize the sterility testing process is essential. These innovations would not only reduce the burden on laboratory personnel but also significantly enhance the accuracy and reliability of results, ultimately improving patient safety and treatment efficacy.

### Summary of the invention

The above-identified objects are solved by the technical teaching as provided with the present invention.

In a first aspect there is provided a cartridge (1a, 1b, 1c) for performing a sequence of steps of an nucleic acid amplification test on a sample, comprising: (i) at least one compartment (2) for receiving the sample; (ii) at least one lysis compartment (3); (iii) at least one nucleic acid purification compartment (4); (iv) at least one compartment (5) comprising a set of nucleic acid amplification reagents, and (v) optionally: at least one waste compartment (6); wherein the cartridge (1a, 1b, 1c), the compartments and all reagents are provided nucleic acid contamination-free; wherein the cartridge (1a, 1b, 1c) is configured to be operable by a device allowing an automated execution of sample preparation and a nucleic acid amplification test, preferably obtaining a qualitative or quantitative test result; wherein the cartridge (1a, 1b, 1c) allows nucleic acid contamination-free fluid transfer between different, or all, compartments comprised; optionally: wherein two or more compartments are physically identical.

One embodiment relates to the cartridge (1a, 1b, 1c) according to the first aspect, wherein the cartridge, the compartments and all reagents are nucleic acid contamination-free as determined by having a Ct value of at least 35, preferably a Ct value of at least 40 for a respective assay target.

One embodiment relates to the cartridge (1a, 1b, 1c) according to the first aspect and the embodiment thereof, wherein the sample is (i) a pharmaceutical sample; or (ii) a biological specimen with a final cellular concentration of at least 0.5 x 10⁶ cells per mL, more preferably of at least 1 x 10⁶ cells per mL, most preferably of at least 10 x 10⁶ cells per mL. One embodiment relates to the cartridge (1a, 1b, 1c) according to the first aspect and any embodiment thereof, wherein the lysis compartment (3) comprises at least one chaotropic agent, preferably guanidinium salt such as guanidine hydrochloride, in a final concentration range of 0.1 to 8 M, preferably 0.2 to 7 M, more preferably 0.5 to 6 M, preferably at least one protease with a final concentration range of 10 to 2,000 µg/mL, preferably 20 to 1,500 µg/mL, more preferably 50 to 1,000 µg/mL, and/or optionally at least one RNase in a final concentration range of 10 to 500 µg/mL, preferably 20 to 300 µg/mL, more preferably 50 to 200 µg/mL, and/or optionally comprises a mechanical disruptor and/or at least one disintegrant supporting lysis of the sample.

One embodiment relates to the cartridge (1a, 1b, 1c) according to the first aspect and any embodiment thereof, wherein the nucleic acid purification compartment (4) comprises a nucleic acid binding material with capacity to process nucleic acids corresponding to at least 100 bacterial / fungi CFU within the nucleic acid background corresponding to at least 0.5 x 10⁶ eukaryotic cells, more preferably at least 1 x 10⁶ eukaryotic cells, most preferably at least 10 x 10⁶ eukaryotic cells, optionally, at least one reconstitution buffer, at least one binding buffer, at least one wash buffer and at least one elution buffer.

One embodiment relates to the cartridge (1a, 1b, 1c) according to the first aspect and any embodiment thereof, wherein the set of nucleic acid amplification reagents comprises at least one nucleic acid amplification buffer, optionally an RT polymerase, at least one DNA polymerase, a set of primers, an internal control sample, and at least one intercalating dye and/or at least one molecular probe.

One embodiment relates to the cartridge (1a, 1b, 1c) according to the first aspect and any embodiment thereof, wherein nucleic acid amplification test detects at least 90%, preferably at least 94% of all known bacterial species and/or at least 30%, preferably at least 37%, of all known fungal species, including all clinically relevant bacterial and/or fungal species.

One embodiment relates to the cartridge (1a, 1b, 1c) according to the first aspect and any embodiment thereof, wherein the at least one compartment (5) additionally comprises a microfluidic device configured to partition a nucleic acid sample into discrete entities suitable for digital nucleic acid amplification and, optionally suitable for obtaining a qualitative or quantitative test result.

A second aspect relates to a use of the cartridge (1a, 1b, 1c) as defined in the first aspect and any embodiment thereof, for performing a nucleic acid amplification test on a sample as defined in the second embodiment of the first aspect.

A third aspect relates to a method of producing the cartridge (1a, 1b, 1c) as defined in the first aspect and any embodiment thereof, wherein assembly of individual components is performed in a nucleic acid-free environment, preferably with DNA depleted components, comprising the following steps: (i) optionally: DNA-depleting all individual and/or pre-assembled components and/or reagents (1a, 1b, 1c); (ii) assembling the housing of the cartridge (1a, 1b, 1c); (ii) inserting into the housing of the cartridge (1a, 1b, 1c), (a) at least one compartment (2) for receiving the sample; (b) at least one lysis compartment (3); (c) at least one nucleic acid purification compartment (4); (d) at least one compartment (5), optionally comprising a set of nucleic acid amplification reagents, and (e) optionally: at least one waste compartment (6); (iii) sealing the cartridge (1a, 1b, 1c), preferably in a nucleic acid contamination-free environment.

A fourth aspect relates to a kit or a kit of parts comprising at least one nucleic acid amplification reagent selected from the group consisting of at least one lysis buffer, at least one binding material, at least one binding buffer, at least one wash buffer, at least one elution buffer, at least one nucleic acid amplification buffer, at least one nucleic acid primer, at least one internal control, optionally at least one RT polymerase, at least one DNA polymerase, at least one intercalating dye and at least one molecular probe;
wherein the kit or the kit of parts is nucleic acid contamination-free; preferably wherein the kit or kit of parts is configured to be operable by or within a cartridge, preferably by or within the cartridge (1a, 1b, 1c) as defined in the first aspect and any embodiment thereof.

One embodiment relates to the kit or the kit of parts according to the fourth aspect, additionally comprising at least one positive control sample.

A fifth aspect relates to a use of the cartridge (1a, 1b, 1c) according to the first aspect and any embodiment thereof, or of the kit or the kit of parts for detecting at least one fungal and/or at least one bacterial contamination in a biological sample with a detection limit of at least 100 CFU, preferably of at least 50 CFU, more preferably of at least 10 CFU.

A sixth aspect relates to a device (7) comprising: (i) the cartridge (1a, 1b, 1c) according to the first aspect and any embodiment thereof; (ii) a detection chamber (8) comprising at least one spectral detector, preferably a fluorescence detector; (iii) a control unit (9) programmed to automate the sequential operation of the cartridge (1a, 1b, 1c) as defined in the first aspect and any embodiment thereof; and (iv) optionally: a computer-implemented system (10) for analysing the signals of a qualitative or quantitative nucleic acid amplification reaction.

A seventh aspect relates to a method of testing the nucleic acid contamination of a sample (2), preferably in an automated manner, using the cartridge (1a, 1b, 1c) according to according to the first aspect and any embodiment thereof, or the kit or kit of parts according to the fourth aspect and any embodiment thereof, wherein the method comprises (i) providing a sample to the compartment (2) for receiving the sample; (ii) allowing lysis of a sample in the lysis compartment (3); (iii) allowing binding of the sample of (ii) to the binding material in the nucleic acid purification compartment (4); (iv) performing at least one wash step and at least one elution step in the nucleic acid purification compartment (4) (v) performing a nucleic acid amplification test in the nucleic acid amplification compartment (5); (vi) optionally: obtaining a qualitative or quantitative test result by detection of a spectral signal in the detection chamber (8) as defined in the sixth aspect; and (vii) optionally: analysing the qualitative or quantitative nucleic acid amplification reaction by the computer implemented system (10) as defined in the sixth aspect.

An eighth aspect relates to a use of the cartridge (1a, 1b, 1c) or of the kit or the kit of parts for determination of the absence of a contamination, preferably in a cell suspension or a pharmaceutical product or a sample thereof, or an alternative aspect relates to a method of screening for a contamination in cartridge (1a, 1b, 1c).

In certain embodiments of the previous aspects the cartridge is utilized in conjunction with an automated data evaluation system. This evaluation processes signals, preferably at least one spectral signal, generated by the nucleic acid amplification test, applying an algorithm that accounts for the assay's positive and negative controls. The application of the algorithm preferably allows to obtain a qualitative and/or quantitative test result. In instances where the internal control (IC) signal is absent, the assay may be automatically deemed invalid, facilitating the detection of potential false-negative results.

In certain embodiments melting curve analysis is employed to differentiate between true positive signals and potential artefacts.

### Brief Description of the Drawings

The following detailed description of the embodiments of the invention will be better understood when read in conjunction with the appended drawings. For the purpose of illustrating the invention, shown in the drawings are embodiments, which are presently exemplified. It should be understood, however, that the invention is not limited to the precise arrangement and instrumentalities of the embodiments shown in the drawings.

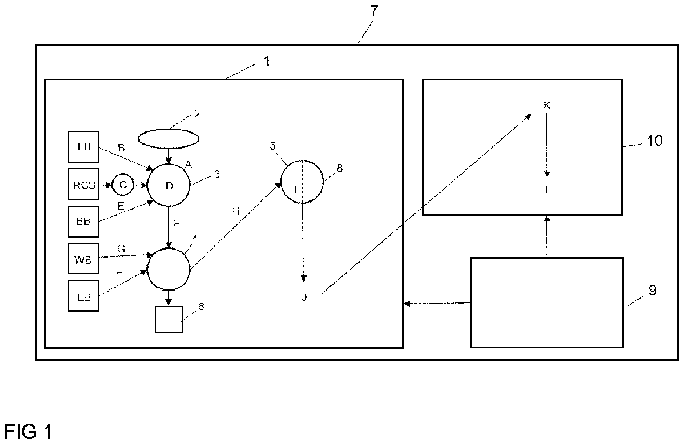

**Figure 1 (FIG 1****)** shows a flow-diagram of a stepwise nucleic acid amplification test inside a cartridge and the embedding of the cartridge (1) into a device (7) for signal detection and computer-implemented analysis. A: Transfer of the sample from the sample compartment (2) to the lysis compartment (3), B: addition of lysis buffer, C: Dissolving the lyophilizate with reconstitution buffer and introduction to the lysis compartment (3), D: Sample lysis, E: Addition of binding buffer (BB), F: Lysate is introduced to the nucleic acid purification compartment (4), G: The nucleic acid binding material is washed with washing buffer, H) DNA is eluted with elution buffer and transferred to nucleic acid amplification compartment (5), I: The eluate is combined with the nucleic acid amplification buffer and reagents, nucleic acid amplification reaction, J: signal detection by the detection chamber (8), K: Data analysis and L: Result output. The automated stepwise process is controlled by the control unit (9). SC = Sample chamber, LB = Lysis buffer, RCB = Re-constitution buffer, BB = Binding buffer, WB = Wash buffer, EB = Elution buffer.

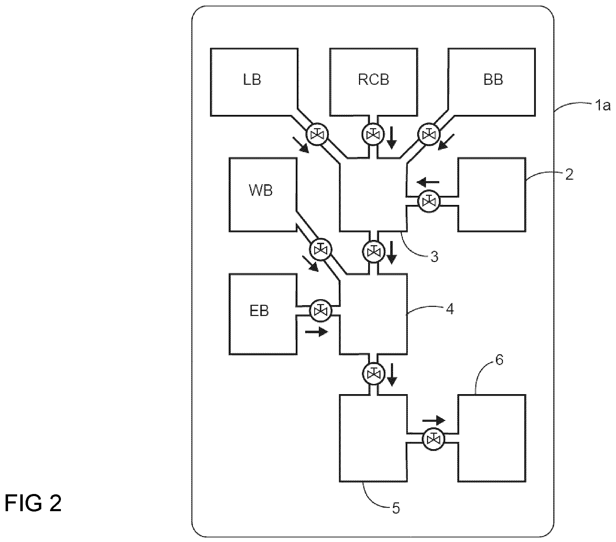

**Figure 2 (FIG 2****):** Schematic drawing of a cartridge (1a), comprising sole storage compartments (LB: Lysis buffer, RCB: Reconstitution buffer, BB: Binding Buffer, WB: Wash buffer, EB: Elution buffer) and respective controllable fluid connections to the reaction compartments: sample compartment (2), lysis compartment (3), nucleic acid purification compartment (4), nucleic acid amplification compartment (5). The waste compartment (6) configured to store disposed material and fluids.

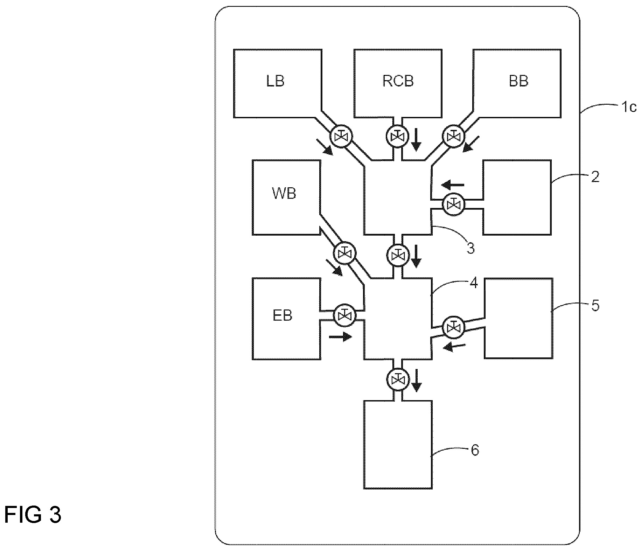

**Figure 3 (FIG 3****):** Schematic drawing of a cartridge (1a), comprising sole storage compartments (LB: Lysis buffer, RCB: Reconstitution buffer, BB: Binding Buffer, WB: Wash buffer, EB: Elution buffer) and respective controllable fluid connections to the reaction compartments: sample compartment (2), lysis compartment (3), nucleic acid purification compartment (4), nucleic acid amplification compartment (5). The waste compartment (6) is configured to store disposed material and fluids.

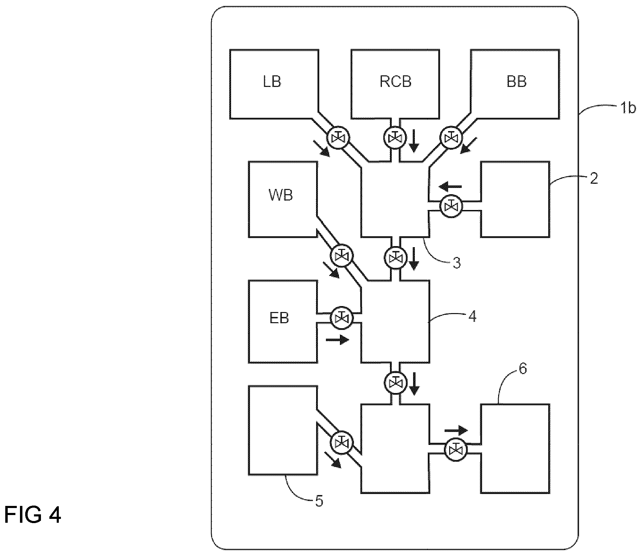

**Figure 4 (FIG 4****):** Schematic drawing of a cartridge (1a), comprising sole storage compartments (LB: Lysis buffer, RCB: Reconstitution buffer, BB: Binding Buffer, WB: Wash buffer, EB: Elution buffer) and respective controllable fluid connections to the reaction compartments: sample compartment (2), lysis compartment (3), nucleic acid purification compartment (4), nucleic acid amplification compartment (5). The waste compartment (6) is configured to store disposed material and fluids.

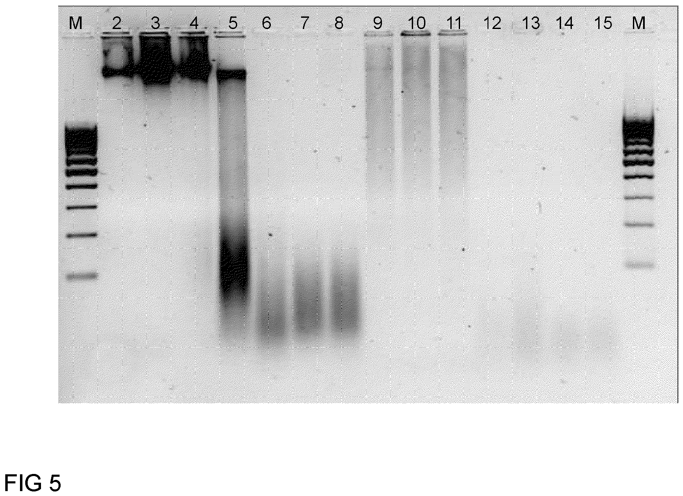

**Figure 5 (FIG 5****):** Resulting agarose gel from **Example 1.** Lane 1: Marker (100 bp ladder), Lanes 2, 3, and 4: *E. coli* DNA without ETO fumigation, Lanes 5, 6, 7 and 8: ETO-treated *E. coli* DNA. Lanes 9, 10, 11: E. *coli* DNA after piperidine treatment. Lanes 12 to 15: E. *coli* DNA after ETO fumigation and piperidine treatment.
**FIG 6 to 17** show the Limit of Detection at 95% confidence level (LOD) for various tested bacterial species as described in **Example 2.** The respective mean Ct value is based on four replicates.
**Figure 6 (FIG 6****):** LOD for *Bacillus subtilis*
**Figure 7 (FIG 7****):** LOD for *Pseudomonas aeruginosa*
**Figure 8 (FIG 8****):** LOD for *Clostridium sporogenes*
**Figure 9 (FIG 9****):** LOD for *Staphylococcus aureus*
**Figure 10 (FIG 10****):** LOD for *Streptococcus pyogenes*
**Figure 11 (FIG 11****):** LOD for *Bacteroides vulgatus*
**Figure 12 (FIG 12****):** LOD for *Escherichia coli*
**Figure 13 (FIG 13****):** LOD for *Pseudomonas protegens*
**Figure 14 (FIG 14****):** LOD for *Bacillus cereus*
**Figure 15 (FIG 15****):** LOD for *Enterococcus fecalis*
**Figure 16 (FIG 16****):** LOD for *Kocuria rhizophila*
**Figure 17 (FIG 17****):** LOD for *Staphylococcus epidermis*

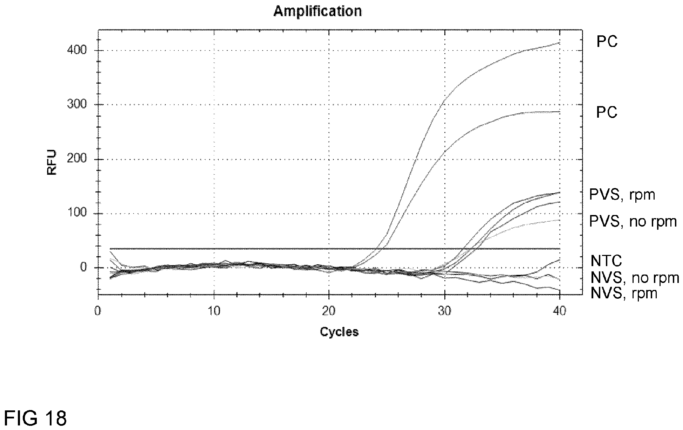

**Figure 18 (FIG 18****):** Amplification curves from **Example 4.** Sample preparation was started from 1 mL of 19 x 10⁶ c/mL CHO cells. CFU standard was the 99 CFU Microsart^{®} Validation Standard of B. *subtilis.* Positive Control (PC, 2 replica), sample with positive validation standard (PVS) and lysis at 1500 rpm (PVS rpm, 3 replica), sample with PVS and lysis without 1500 rpm (PVS no rpm, 1 replica), sample with negative validation standard, NVS, and lysis at 1500 rpm (NVS rpm, 1 replica), sample with NVS and lysis without 1500 rpm (NVS no rpm, 1 replica), no template control (NTC, 2 replica). Threshold: horizontal bar.

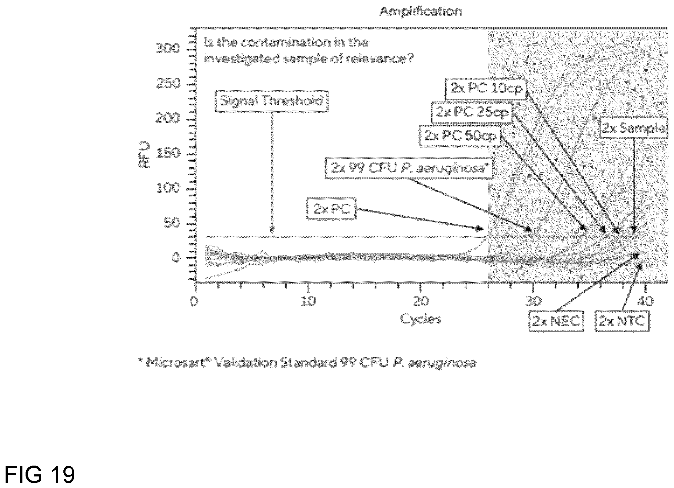

**Figure 19 (FIG 19****):** The Microsart^{®} ATMP Bacteria real-time PCR assay demonstrates an absence of contaminating bacterial DNA, resulting in no significant signal (Ct ≥ 40) during 40 amplification cycles for non-spiked reactions. The high purity of the PCR reaction chemistry enables the detection of as few as 10 template copies, producing a Ct value below 40. Additionally, the assay is capable of detecting as low as 5 template copies within a PCR reaction. CFU: colony forming unit, cp: copies, NEC: negative extraction control, NTC: no template control, PC: positive control, RFU: relative fluorescence units.
**Figure 20 (FIG 20****):** Average Ct values as a function of the 16S rRNA gene copy number in real-time PCR, incorporating a dPCR-quantified positive control (PC) as a standard. A correlation is observed between Ct values and 16S rRNA gene copy numbers, where lower Ct values correspond to higher gene copy numbers. The measurement was conducted with a sample size of N = 24 replicates.

### Detailed Description

The present invention solves the problems in the prior art by providing a nucleic acid contamination-free cartridge for complete and automated nucleic acid amplification testing of sterility in complex biological samples such as cell suspension. This solution offers numerous advantages over traditional bench-top PCR testing kits, significantly enhancing the accuracy, reliability, and efficiency of sterility testing.

By minimizing the risk of external nucleic acid contamination through its closed-system design, the invention ensures that test results are accurate and reliable, preventing false-positive results and maintaining the integrity of sterility testing. Automation of the entire nucleic acid amplification testing process eliminates many common sources of human error associated with manual handling of samples and reagents, leading to more consistent and reproducible results and improving the overall quality of the testing process. Thus, one key advantage of nucleic acid amplification based sterility testing is the very short time from testing to results, allowing for the immediate use of a medical product for the patient's benefit. Another advantage is the broad detection of potential contaminants, which cannot be achieved with the classical growth-based approach due to the limitations of media and culture conditions.

By streamlining the testing process and reducing the need for skilled personnel and consumables, the invention lowers the time-to-result, combined with an increased robustness and convenience. The use of a standardized, automated system ensures uniformity in testing procedures, which is essential for meeting the rising standards of pharmaceutical quality control.

In summary, the nucleic acid contamination-free cartridge for nucleic acid amplification testing addresses the critical challenges of traditional sterility testing methods, offering a superior solution that improves accuracy, efficiency, and patient safety.

In a first aspect there is thus provided a cartridge (1a, 1b, 1c) for performing a sequence of steps of an nucleic acid amplification test on a sample, comprising: (i) at least one compartment (2) for receiving the sample; (ii) at least one lysis compartment (3); (iii) at least one nucleic acid purification compartment (4); (iv) at least one compartment (5) comprising a set of nucleic acid amplification reagents, and (v) optionally: at least one waste compartment (6); wherein the cartridge (1a, 1b, 1c), the compartments and all reagents are provided nucleic acid contamination-free; wherein the cartridge (1a, 1b, 1c) is configured to be operable by a device allowing an automated execution of sample preparation and a nucleic acid amplification test, preferably obtaining a qualitative or quantitative test result; wherein the cartridge (1a, 1b, 1c) allows nucleic acid contamination-free fluid transfer between different, or all, compartments comprised; optionally: wherein two or more compartments are physically identical.

In the present invention, the term "free of contaminating nucleic acids" or "nucleic acid contamination-free" is defined as the condition where no unintended nucleic acids (any kind of DNA or RNA) are detectable in a sample and its components when tested using a pan-bacterial and pan-fungal real-time PCR assay, such as the Microsart^{®} ATMP Sterile Release Detection Kit, or a real-time RT-PCR assay, or related or comparable products, such as the Applied Biosystems^{™} SteriSEQ^{™} Rapid Sterility Testing Kit from Thermo Fisher Scientific. Specifically, this condition is met when the cycle threshold (Ct) value in real-time PCR exceeds 40 for a non-spiked reaction, and 10 template copies, preferably 5 template copies, can be detected with a Ct value below 40, as shown in FIG 19. These Ct thresholds may vary when using products other than the Microsart^{®} ATMP Sterile Release Detection Kit or comparable alternatives, when the test is performed in non-standardized real-time PCR tubes or reaction chambers, or when the reaction volume deviates from the recommendations in the Microsart^{®} ATMP Sterile Release Detection Kit manual.

FIG 19 shows results of the Microsart^{®} ATMP Bacteria real-time PCR assay, which was specifically designed to be free of contaminating bacterial DNA, ensuring no detectable signal (Ct ≥ 40) during 40 amplification cycles in non-spiked reactions. In real-time PCR, a non-spiked reaction tests the sample without the addition of external nucleic acids, assessing for the presence of any naturally occurring contaminants. A high or undetectable Ct value (Ct ≥ 40) in these conditions confirms the absence of contamination in the assay or the sample.

In a spiked reaction, a defined quantity of bacterial nucleic acids is introduced to assess the assay's sensitivity and validate its ability to detect the target nucleic acids accurately. A lower Ct value, typically below 40, in the spiked reaction indicates proper assay performance and confirms its capability to detect bacterial DNA at low concentrations.

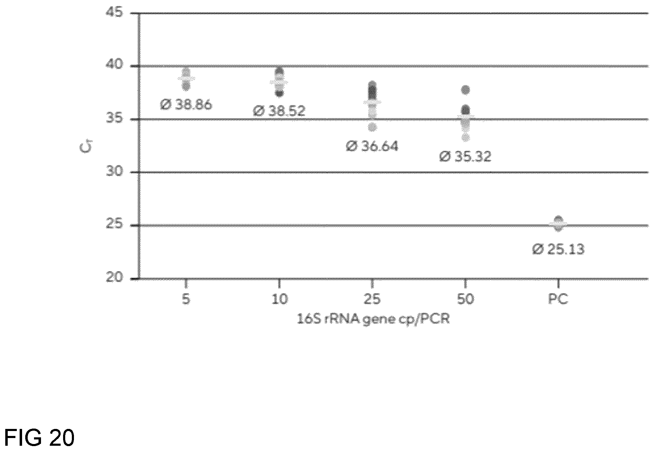

The high purity of the Microsart^{®} ATMP PCR reaction chemistry allows the detection of as few as 10 template copies, leading to a detectable Ct value below 40. In some cases, the assay can even detect as few as 5 template copies in the reaction, as shown in FIG 20. Together, the spiked and non-spiked reactions provide a comprehensive validation, confirming both the absence of contamination and the assay's high sensitivity for bacterial DNA detection.

In a nucleic-acid contamination free environment, such as a cartridge, a reagent or a buffer, any detected and targeted nucleic acid originates solely from the sample, without measurable interference from external or residual nucleic acid contamination. Thus, "contamination" refers to a measurable and detectable presence of nucleic acids (DNA/RNA) or fragments thereof that are targeted and detectable by a given nucleic acid amplification test using reagents and reaction conditions allowing the maximum sensitivity possible according to the state of the art.

A flow-diagram illustrating the subsequent steps of PCR sterility testing in cartridge and the embedding of said cartridge in device (7), comprising a computer-implemented qualitative and quantitative analysis of nucleic acid contamination, is shown in **FIG 1****.**

The provided cartridge (1a, 1b, 1c) is designed to perform a nucleic acid amplification test on a sample through a series of precisely controlled and automated steps, ensuring nucleic acid contamination-free handling and accurate sterility test. The cartridge (1a, 1b, 1c) effectively integrates multiple compartments for sample reception, lysis, purification, amplification, and waste management, ensuring a streamlined and contamination-free process. This automated system is designed to provide reliable and precise nucleic acid amplification test results, meeting the high standards required in clinical and pharmaceutical research settings.

Preferably, the cartridge is manufactured in a controlled DNA-free environment using DNA-depleted components and buffers. A strictly controlled assembly under highly pure conditions may allow for bypassing the need for terminal DNA depletion/sterilization of the final cartridge product. This approach eliminates the risk of damaging the enzymes used in the cartridge while simultaneously minimizing the risk of nucleic acid contamination.

The cartridge may comprise at least two reaction compartments, specifically at least one lysis compartment (3) and at least one nucleic acid purification compartment (4), a plurality of storage compartments comprising the at least one lysis buffer (LB), a reconstitution buffer (RCB), a binding buffer (BB), at least one wash buffer (WB) and at least one elution buffer (EB). The contents of each storage compartment may be introduced into the at least one lysis compartment (3) or the nucleic acid purification compartment (4) compartment in a manner controlling the flow by e.g., an automatically conducted slider mechanism. The arrangement and number of reaction chambers may vary.

Preferably, all buffers are DNA-depleted and provided in lyophilized or liquid form within the cartridge.

In certain embodiments, reconstitution of the lyophilized material may be performed by addition of reconstitution buffer.

A lysis buffer is designed to break open cells, releasing their internal contents, including proteins, DNA, RNA, and other biomolecules. It may contain at least one disintegrant to disrupt membranes (esterases, detergents, etc.), enzymes to degrade proteins (e.g. proteinase) and other biomolecules to protect the integrity of the nucleic acid molecules of interest during lysis (e.g. Ethylenediaminetetraacetic acid (EDTA)). The skilled person is well aware of the fact that a lysis buffer may need adaptions to effectively and efficiently lyse all target cells of interest.

A reconstitution buffer is usually specifically formulated to dissolve or rehydrate lyophilized proteins, peptides, or nucleic acids. It ensures the reconstituted molecules maintain their structural integrity and functional activity by providing an optimal pH and ionic strength.

A binding buffer may be used to promote the interaction between the nucleic acids and the binding material. This buffer typically contains salts (sodium, potassium, guanidium salts etc.) and other components (polyethylene polymers, urea etc.) at a specific pH to facilitate the specific binding of the target molecules while minimizing non-specific interactions.

**FIG 2** to **4** schematically illustrate optional embodiments of the arrangement of storage and reaction compartments and their fluidic connections, but any other type of arrangement or mechanical configuration that is suitable for performing the subsequent steps illustrated in the flow-diagram of **FIG 1** may be used.

In one embodiment the nucleic acid purification and nucleic acid amplification may both be performed in one compartment, such as the nucleic acid purification compartment (4), as displayed in **FIG 3****.**

The skilled person will readily understand that the architectures and schemes shown in **FIG 1** to **FIG 4** are exemplary schemes and configurations and that different architectures and varying flow schemes may be applied provided that the high degree of sterility relevant for processing and a compatible architecture of the cartridge and the testing device is guaranteed.

In the context of the present invention, the term "compartment" is to be understood broadly. Therefore, a compartment may be understood as one physically distinct entity, or it may be understood as reaction compartment allowing the function of different compartments in one reaction room. Multiple compartments according to the first aspect, particularly the at least one compartment (2) for receiving the sample; the at least one lysis compartment (3); the at least one nucleic acid purification compartment (4) and/or the at least one compartment (5) comprising a set of nucleic acid amplification reagents may either be physically distinct entities or represent a single compartment, wherein receiving of the sample, lysis nucleic acid purification, and amplification is accommodated. Alternatively, processes designated as occurring within a single compartment may be distributed across several compartments. Any configuration of compartmentalization that enables the sequential execution of sample lysis, nucleic acid purification, and amplification is encompassed within the scope of the invention.

A "reaction chamber" according to the present invention is to be understood as any enclosed or partially enclosed space designed to facilitate chemical and/or biochemical reactions, preferably the nucleic acid purification and facilitation of nucleic acid amplification tests. Said reaction chamber may take various forms, including a simple confined space within a cartridge, or more complex configurations such as beads, microparticles, microcapsules, or droplets, each serving as discrete microenvironments for controlled chemical and/or biochemical processes.

The at least one reaction chamber can be present within one or more compartment(s) according to the present invention.

One or more reaction chamber(s) may be housed in a single compartment, allowing for parallel or sequential reactions, or they may be distributed across multiple compartments to achieve desired reaction processes. Advantageously, this compartmentalized architecture supports complex workflows, such as sample lysis, nucleic acid purification and/or nucleic acid amplification tests, either within a single compartment or across multiple interconnected compartments.

In certain embodiments, the testing procedure begins with the sample being introduced into the cartridge through the designated sample compartment (2). This compartment is specifically designed to securely hold the sample and prevent any contamination from external sources. Once the sample is securely in place, it is transferred to the at least one lysis compartment (3). In certain embodiments, sample chamber and lysis compartment may be identical. In this step, the cells within the sample are lysed to release nucleic acids. Lysis buffer and/or at least one disintegrant may be stored ab initio in the at least one lysis compartment (3) or are introduced in a controlled manner from a storage chamber containing the lysis buffer and/or at least one disintegrant. Subsequently, the lysed cells are introduced to the nucleic acid purification compartment (4). After at least one washing steps, the nucleic acids are eluted from the nucleic acid binding material and introduced into the at least one nucleic acid amplification compartment (**FIG 2**). Alternatively, nucleic acid amplification reagents may be performed in the nucleic acid purification compartment (4) on the nucleic acid binding material (**FIG 3**). Another alternative is the separate storage of nucleic acid amplification reagents and their controlled introduction into the at least one nucleic acid amplification compartment (**FIG 4**). Optionally, any excess reaction mixtures may be disposed into the waste compartment (6).

The compartment contains reagents and enzymes necessary for the lysis process, which are nucleic acid contamination-free, ensuring that no external nucleic acids interfere with the sample.

After lysis, the lysate is moved to the nucleic acid purification compartment (4). This compartment facilitates the separation of nucleic acids from other cellular components and impurities. It typically involves binding the nucleic acids to a solid-phase medium, washing away contaminants, and then eluting pure nucleic acids. This step is crucial for obtaining high-quality nucleic acids necessary for accurate amplification and testing.

The purified nucleic acids are then transferred to the amplification compartment (5), which contains a set of nucleic acid amplification reagents. These nucleic acid amplification reagents include primers, nucleotides, polymerase enzymes, and any necessary buffers. The amplification process exponentially increases the amount of target nucleic acid, making it detectable and measurable.

Optionally, during the process, waste materials generated from lysis and purification are directed to the waste compartment (6). This compartment securely contains the waste products, aiding in removal buffers and cellular debris from the reaction compartments.

In preferred embodiments, the cartridge is designed to be operated by a device that automates the execution of each step, from sample preparation to nucleic acid amplification. This device ensures precise and contamination-free fluid transfer between compartments, reducing the risk of human error and enhancing the reliability of the results.

The cartridge is configured to allow the detection of an intercalating dye or molecular probe. Depending on the employed algorithm, the device (7) may provide qualitative results (presence or absence of the target nucleic acid(s)) or quantitative results (amount of target nucleic acid(s)) based on the received signal from the intercalating dye or molecular probe.

One embodiment relates to the cartridge (1a, 1b, 1c) according to the present invention, wherein the cartridge, the compartments and all reagents are free of contaminating nucleic acid as defined previously, in particular as determined by having a Ct value of at least 35, preferably a Ct value of at least 40 for a respective assay target.

One embodiment relates to the cartridge (1a, 1b, 1c) according to the present invention, wherein the cartridge, the compartments and all reagents are nucleic acid contamination-free as determined by having a Ct value of at least 35, 36, 37, 38, 39 or preferably at least 40, or above, for example, at least 41, 42, 43, 44 or at least 45, for a respective assay target.

In nucleic acid amplification tests, such as PCR or isothermal nucleic acid amplification, maintaining a nucleic acid contamination-free environment is crucial. The presence of contaminant nucleic acids can lead to false-positive results, which may comprise the accuracy and reliability of the tests. One of the critical measures of PCR efficiency and reliability is the Cycle threshold (Ct) value, which indicates the cycle number at which a detection signal representing the amplified products exceeds the background level and becomes detectable. Contamination with extraneous nucleic acids can cause premature amplification, leading to lower (earlier) Ct values. This can falsely indicate a higher initial quantity of target nucleic acid, misguiding outcomes. For instance, in a sterility test, a false-positive result due to contamination might incorrectly suggest the presence of microbial DNA, leading to unnecessary interventions or erroneous conclusions. Inconsistent contamination levels can cause significant variability in Ct values across different tests or samples. This variability undermines the reproducibility of results. High sensitivity and specificity are hallmarks of effective nucleic acid amplification tests. Contaminants can compromise both, leading to false positives (reduced specificity) and potentially masking the presence of low-abundance targets (reduced sensitivity). Ensuring a nucleic acid contamination-free process preserves the test's integrity, where Ct values accurately represent the target nucleic acid's presence and quantity.

One embodiment relates to the cartridge (1a, 1b, 1c) according to the according to the present invention, wherein the sample is (i) a pharmaceutical sample; or (ii) a biological specimen with a final cellular concentration of at least 0.5 x 10⁶ cells per mL, more preferably of at least 1 x 10⁶ cells per mL, most preferably of at least 10 × 10⁶ cells per mL.

In the present invention a pharmaceutical sample refers to a formulated or processed material intended for therapeutic use, typically comprising active pharmaceutical ingredients (APIs) and excipients, but excluding any living cells or cellular components.

Advantageously, the cartridge allows the processing of cell-dense samples in a volume of up to 5 mL, preferably up to 1.5 mL, more preferably up to 1 mL, which enhances the probability of detecting nucleic acid contaminants, thereby improving the diagnostic sensitivity of the assay.

In certain embodiments the cartridge allows the processing of cell-dense samples in a volume of at least 100 µl, at least 200 µl, at least 300 µl, at least 400 µl, at least 500 µl, at least 600 µl at least 700 µl, at least 800 µl, at least 900 µl or at least 1 mL and/or up to 1 mL, up to 1.1 mL, up to 1.2 mL, up to 1.3 mL, up to 1.4 mL, up to 1.5 mL, up to 1.6 mL, up to 1.7 mL, up to 1.8 mL, up to 1.9 mL, up to 2 mL, up to 2.1 mL, up to 2.2 mL, up to 2.3 mL, up to 2.4 mL, up to 2.5 mL, up to 2.6 mL, up to 2.7 mL, up to 2.8 mL, up to 2.9 mL, up to 3 mL, up to 3.1 mL, up to 3.2 mL, up to 3.3 mL, up to 3.4 mL, up to 3.5 mL, up to 3.6 mL, up to 3.7 mL, up to 3.8 mL, up to 3.9 mL, up to 4 mL, up to 4.1 mL, up to 4.2 mL, up to 4.3 mL, up to 4.4 mL, up to 4.5 mL, up to 4.6 mL, up to 4.7 mL, up to 4.8 mL, up to 4.9 mL or up to 5 mL.

One embodiment relates to the cartridge (1a, 1b, 1c) according to the present invention, wherein the lysis compartment (3) comprises at least one chaotropic agent, preferably guanidinium salts such as guanidine hydrochloride, in a final concentration range of 0.1 to 8 M, preferably 0.2 to 7 M, more preferably 0.5 to 6 M, preferably at least one protease with a final concentration range of 10 to 2,000 µg/mL, preferably 20 to 1,500 µg/mL, more preferably 50 to 1,000 µg/mL, and/or optionally at least one RNase in a final concentration range of 10 to 500 µg/mL, preferably 20 to 300 µg/mL, more preferably 50 to 200 µg/mL, and/or optionally comprises a mechanical disruptor and/or at least one disintegrant supporting lysis of the sample.

Lysing biological samples presents several challenges, which require specific methods to effectively disrupt cells aiming at resolving nucleic acids.

In cell lysis, a variety of disintegrants are known in the art to efficiently break down cell structures and release cellular components. Detergents such as SDS, Triton X-100, and NP-40 are used to solubilize lipids and proteins, effectively disrupting cell membranes. Enzymes, including lysozyme, protease, and DNase, target specific cellular components, aiding in the degradation of cell walls, proteins, and DNA, respectively. Salts like ammonium sulfate and sodium chloride can induce cell lysis through osmotic shock by altering cellular osmolarity. Additionally, chaotropic agents such as urea and guanidium salts, such as guanidinium chloride, disrupt molecular interactions and denature biomolecules.

In certain embodiments, the at least one lysis compartment (3) may be suitable for mechanical disruption, wherein mechanical disruption is achieved through an automatically controlled homogenization process, wherein the homogenization process involves applying mechanical forces, such as sonication, bead milling, pneumatic shearing and electroporation, but not limited thereof.

Preferably, the at least one lysis compartment (3) is specifically designed to accommodate a variety of biological samples, incorporating adjustable speed settings and customizable impact levels to optimize the disruption efficiency while minimizing damage to nucleic acids.

In certain embodiments, the at least one lysis compartment (3) may be suitable for enzymatic digestion, preferably wherein enzymatic digestion is facilitated through the controlled introduction of at least one lytic enzyme such as, but not limited to, at least one protease, at least one collagenase and at least one lysozyme.

Preferably, precise reaction conditions such as temperature and pH are maintained in the at least one lysis compartment (3) for optimal enzymatic activity.

The at least one lysis compartment may include at least one sensor to monitor the digestion process in real-time, and features adjustable parameters to fine-tune the enzyme activity according to the specific requirements of the biological sample.

In the lysis compartment, chemical lysis may be performed using at least one detergent, such as, but not limited to, SDS and Triton X-100, or at least one chaotropic agent, such as guanidinium chloride.

In certain embodiments the at least one disintegrants is stored in a form stable at room temperature, preferably in combination with at least one other, preferably more than one other, disintegrant in the form of a lysis buffer.

In certain embodiments, the chemicals and enzymes for lysis are stored in at least one separate storage compartment and may be introduced into the at least one lysis compartment (3) in a controlled manner.

One embodiment relates to the cartridge (1a, 1b, 1c) according to the present invention, wherein the nucleic acid purification compartment (4) comprises a nucleic acid binding material with capacity to process nucleic acids corresponding to at least 100 bacterial / fungi CFU within the nucleic acid background corresponding to at least 0.5 x 10⁶ eukaryotic cells, more preferably at least 1 x 10⁶ eukaryotic cells, most preferably at least 10 x 10⁶ eukaryotic cells, optionally, at least one reconstitution buffer, at least one binding buffer, at least one wash buffer and at least one elution buffer.

One embodiment relates to the cartridge (1a, 1b, 1c) according to the present invention, wherein the nucleic acid purification compartment (4) comprises a nucleic acid binding material with capacity to process nucleic acids corresponding to at least 100 bacterial / fungi CFU within the nucleic acid background corresponding to at least 0.5 x 10⁶ eukaryotic cells, more preferably at least 1 × 10⁶ eukaryotic cells, most preferably at least 10 x 10⁶ eukaryotic cells or an equivalent quantity of non-target nucleic acids optionally, at least one reconstitution buffer, at least one binding buffer, at least one wash buffer and at least one elution buffer.

In a nucleic acid amplification reaction, the nucleic acid binding material typically refers to the solid phase used to capture or immobilize nucleic acids during various stages of nucleic acid purification. These materials have a high affinity for DNA or RNA and are used to selectively bind nucleic acids from a sample mixture, allowing for the washing away of contaminants before elution and subsequent amplification. These materials rely on different mechanisms such as charge interactions, hydrogen bonding, or specific ligand-receptor interactions to bind nucleic acids.

In certain embodiments the primers may hybridize to a fungal and/or bacterial ribosomal RNA genes, e.g., 16S rRNA gene and/or 18S rRNA gene.

Nucleic acid amplification is achieved through an automated process incorporating techniques such as, but not limited to, polymerase chain reaction (PCR), isothermal amplification or transcription-mediated amplification by a reverse transcriptase.

Preferably, the nucleic acid purification compartment (4) is specifically designed to handle a variety of sample types and volumes, incorporating precise thermal cycling capabilities and real-time monitoring systems to optimize amplification efficiency and accuracy.

Preferably, the nucleic acid purification compartment (4) is configured to facilitate rapid thermal transitions essential for efficient DNA or RNA amplification. This includes heating and cooling systems that allow for quick temperature changes necessary for the heating, denaturation, annealing, and extension phases of PCR, or the constant temperature needed for isothermal methods.

In certain embodiments, the nucleic acid purification compartment (4) may maintain precise control over reaction conditions such as temperature, ion concentration, and pH to ensure optimal activity of a DNA polymerase or optionally, a reverse transcriptase.

Preferably, the nucleic acid purification compartment may include at least one sensor to monitor nucleic acid amplification conditions in real-time, with adjustable parameters that may be fine-tuned according to the specific requirements of a respective nucleic acid amplification process.

In certain embodiments, reagents for amplification may be stored in at least one separate, controlled environment storage compartment and introduced into the nucleic acid compartment (4) in a precise, automated manner to further enhance reproducibility and reliability of the nucleic acid amplification.

In certain embodiments, the nucleic acid binding material may be silica beads, magnetic beads, cellulose-based membranes, anion exchange resins, polymer-based resins, nylon membranes, glass fibers, or any material known in the art reversibly or irreversibly binding nucleic acid molecules, depending on the purification methodology.

Nucleic acid amplification is achieved through an automated process incorporating techniques such as, but not limited to, polymerase chain reaction (PCR), isothermal amplification, or transcription-mediated amplification. Preferably, the nucleic acid compartment (4) is specifically designed to handle a variety of sample types and volumes, incorporating precise thermal cycling capabilities and real-time monitoring systems to optimize amplification efficiency and accuracy. The nucleic acid compartment (4) may be equipped to facilitate rapid thermal transitions essential for efficient DNA or RNA amplification. This includes advanced heating and cooling systems that allow for quick temperature changes necessary for the denaturation, annealing, and extension phases of PCR, or the constant temperature needed for isothermal methods.

In certain embodiments, the nucleic acid purification compartment (4) may include at least one sensor to monitor these conditions in real-time, with adjustable parameters that can be fine-tuned according to the specific requirements of the nucleic acid amplification process.

One embodiment relates to the cartridge (1a, 1b, 1c) according to the present invention, wherein the set of nucleic acid amplification reagents comprises at least one nucleic acid amplification buffer, optionally an RT polymerase, at least one DNA polymerase, a set of primers, an internal control sample, and at least one intercalating dye and/or at least one molecular probe.

Preferably, the at least one nucleic acid amplification buffer and the set of nucleic acid amplification reagents is DNA-depleted and may be provided in lyophilized or liquid form in a storage compartment (5). The reconstitution of the lyophilized material may be carried out directly using the eluate or by addition of reconstitution buffer.

In certain embodiments the nucleic acid amplification reagents are stored in stable form or optionally dissolved in nucleic acid purification buffer at room temperature.

In certain embodiments the nucleic acid amplification reagents are stored in stable form or optionally dissolved in nucleic acid purification buffer in a temperature range of 2° C to 8° C.

Real-time PCR (real-time PCR) is a highly sensitive technique that allows for the amplification and simultaneous quantification of nucleic acids. It has become a gold standard in molecular diagnostics, particularly for detecting and quantifying viral and microbial DNA and RNA. The process involves the use of fluorescent dyes or probes that emit fluorescence in response to the accumulation of PCR product during each amplification cycle. This fluorescence is measured in real-time, providing quantitative data on the amount of target nucleic acid present in the sample. Real-time PCR is distinguished from traditional PCR by its ability to monitor the amplification process as it occurs, rather than only at the end. This is achieved through the use of various chemistries, including DNA-binding dyes like SYBR Green, or more specific probe-based systems such as TaqMan probes, molecular beacons, and others. These detection systems enable real-time PCR to be both highly sensitive and specific, capable of distinguishing between closely related genetic sequences (Mackay et al., Real-time PCR in virology, Nucleic Acids Research (2002), 30, 6, 1292-1305).

In certain embodiments, the nucleic acid amplification reagents for amplification may be stored in at least one compartment (5) comprising a set of nucleic acid amplification reagents and introduced into the nucleic acid compartment (4) in a controllable manner.

In certain embodiments, the intercalating dye may be SYTO-9, SYTO-13, SYTO-16, SYTO-60, SYTO-62, SYTO-64, SYTO-82, POPO-3, TOTO-3, BOBO-3, TO-PRO-3, YO-PRO-1, SYTOX Orange, SYBR Green I, EvaGreen and/or PicoGreen, or any combination thereof, but not limited thereof.

In certain embodiment, the molecular probe may be a TaqMan probe, a molecular beacon, a FRET probe, a scorpion probe and/or a set of dual hybridization probes, or any combination thereof, but not limited thereof.

In certain embodiments, a combination of at least on intercalating dye and at least one molecular probe is used to measure multiple genomic targets in a single nucleic acid amplification process.

One embodiment relates to the cartridge (1a, 1b, 1c) according to the present invention, wherein nucleic acid amplification test detects at least 90%, preferably at least 94% of all known bacterial species and/or at least 30%, preferably at least 37%, of all known fungal species, including all clinically relevant bacterial and/or fungal species.

Preferably, the term "all known bacterial and fungal species" refers to any bacterial and fungal species that are catalogued, indexed, or otherwise accessible in relevant repositories or databases, such as those maintained by recognized scientific institutions or organizations. This term also encompasses any species that can be detected or identified using detection methods and technologies that are established and known to a person skilled in the art. The scope of "all known" includes species that have been characterized in scientific literature, stored in biological collections, or are identifiable through genomic, phenotypic, or molecular techniques. A preferable database and two search settings are listed below.

### Database:

SILVA ribosomal RNA database project
https://www.arb-silva.de/

### Database version:

SILVA SSU and LSU databases 132 as of Dec 13, 2017
Subset: SSU Ref NR 99
https://www.arb-silva.de/documentation/release-132/

### Search tools:

TestPrime https://www.arb-silva.de/search/testprime/
Settings:
   Maximum Number of mismatches = 0, 1, 2 or 3, respectively
   Length of 0-mismatch zone at 3' end = 2
TestProbe https://www.arb-silva.de/search/testprobe/
Settings:
   Maximum Number of mismatches = 0, 1, 2 or 3, respectively

One embodiment relates to the cartridge (1a, 1b, 1c) according to the present invention, wherein nucleic acid amplification test detects at least 90%, 91%, 92%, 93%, 94%, of all known bacterial species and/or at least 30%, 31%, 32%, 33%, 34%, 35%, 36%, 37%, of all known fungal species, including all clinically relevant bacterial and/or fungal species.

Clinically relevant bacterial and/or fungal species are documented in the European Pharmacopoeia (11^{th} edition) in sections EP 2.6.1, EP 2.6.7 and EP 2.6.27, and all species listed in in Table 4 of Example 5.

One embodiment relates to the cartridge (1a, 1b, 1c) according to the present invention, wherein the at least one compartment (5) additionally comprises a microfluidic device configured to partition a nucleic acid sample into discrete entities suitable for digital nucleic acid amplification and, optionally suitable for obtaining a qualitative or quantitative test result.

Partitioning of a nucleic acid sample enables Digital PCR (dPCR), which works by partitioning a PCR sample into thousands of individual reactions, each containing either zero or one (or very few) copies of the target DNA or RNA molecule. After amplification, each partition is analyzed to determine the presence or absence of the target sequence. This process results in a binary outcome - each partition is either positive (the target sequence is present) or negative (the target sequence is absent). The number of positive reactions is then counted and, using statistical analysis based on the Poisson distribution, the exact number of target molecules in the original sample can be calculated. Unlike real-time PCR, this method provides absolute quantification without the need for standard curves, making it highly accurate. Digital PCR is particularly valuable in applications that require the detection of low levels of pathogens, rare genetic mutations, or subtle copy number variations.

In certain embodiments, the cartridge (1a, 1b, 1c) allows for a multiplex assay system comprising at least one compartment designed to simultaneously process a plurality of nucleic acid amplification tests. Each nucleic acid amplification test within the compartment may be associated with a specific fluorescent dye to enable individual detection. For example, bacterial, mycoplasma, and fungal contaminants may be identified in a single reaction compartment, preferably in a single reaction chamber, e.g., bacterial signals are detected in the green channel, mycoplasma signals in the yellow channel, fungal signals in the red channel, and the internal control (IC) in the dark red channel. Advantageously, this configuration facilitates a simultaneous analysis within a single reaction compartment, preferably in a single reaction chamber.

In certain embodiments, the multiplex assay system comprises a plurality of compartments within a single cartridge. Each chamber may independently perform a nucleic acid amplification test along with an internal control, thus enabling parallel processing of reactions on the same sample. For instance, separate chambers can perform assays for mycoplasma plus IC, bacteria plus IC, and fungal organisms plus IC, respectively.

In certain embodiments, the cartridge (1a, 1b, 1c) may allow a plurality of nucleic acid amplification tests to occur staggered or at different times.

In certain embodiments, the cartridge (1a, 1b, 1c) may allow for a multiplex assay system with multiple reaction chambers that can analyze several samples in parallel, optionally in one or more than one compartment, or test the same sample redundantly in one or more than one compartment(s).

Multiplexing or a multiplex assay system may refer to the ability to conduct multiple nucleic acid amplification tests simultaneously within a single compartment. Preferably, each assay is individually distinguishable and can be independently analyzed, typically through the use of different spectral signals, such as those emitted from different fluorescent dyes.

Parallel processing relates to the capability of processing a plurality of nucleic acid amplification tests simultaneously within a multiplex assay system, but not necessarily within the same compartment. It may involve conducting the plurality of nucleic acid amplification tests across different compartments, or different tests within the same or different compartments concurrently.

A second aspect relates to a use of the cartridge (1a, 1b, 1c) according to the present invention, for performing a nucleic acid amplification test on a sample according to the present invention.

A third aspect relates to a method of producing the cartridge (1a, 1 b, 1c) according to the present invention, wherein assembly of individual components is performed in a nucleic acid-free environment, preferably with DNA depleted components, comprising the following steps: (i) optionally: DNA-depleting all individual and/or pre-assembled components and/or reagents (1a, 1b, 1c); (ii) assembling the housing of the cartridge (1a, 1b, 1c); (ii) inserting into the housing of the cartridge (1a, 1b, 1c), (a) at least one compartment (2) for receiving the sample; (b) at least one lysis compartment (3); (c) at least one nucleic acid purification compartment (4); (d) at least one compartment (5), optionally comprising a set of nucleic acid amplification reagents, and (e) optionally: at least one waste compartment (6); (iii) sealing the cartridge (1a, 1b, 1c), preferably in a nucleic acid contamination-free environment.

Universal nucleic acid amplification assays are highly susceptible to contamination, with bacterial and fungal DNA being particularly ubiquitous, making universal bacterial or fungal PCR assays especially prone to interfering nucleic acid detection. Therefore, it is crucial to completely eliminate bacterial and fungal DNA from all consumables and reagents to prevent them from generating false-positive signals in PCR.

Depleting contaminating DNA from components and/ or reagents serves to enhance the accuracy and reliability of nucleic acid-based assays. The skilled person is well aware of established DNA depletion methodologies, such as the use of nuclease enzymes (e.g., DNase) for enzymatic degradation of DNA, chlorine dioxide (ClO₂) for chemical decontamination of surfaces, ultrasound for physical disruption and removal of DNA, Sartobind^{®} membrane adsorbers for the capture and removal of DNA contaminants, and Vivaspin^{®} 100 kDa ultrafiltration devices for the concentration and purification of samples, or any combination thereof.

DNA depletion using ethylene oxide (ETO) is described in Example 2. A DNA depletion method must reduce DNA contamination to very small, non-amplifiable fragments, while not inhibiting or interfering with the subsequent process of sample preparation or the ensuing PCR reaction.

A fourth aspect relates to a kit or a kit of parts comprising at least one nucleic acid amplification reagent selected from the group consisting of at least one lysis buffer, at least one binding material, at least one binding buffer, at least one wash buffer, at least one elution buffer, at least one nucleic acid amplification buffer, at least one nucleic acid primer, at least one internal control, optionally at least one RT polymerase, at least one DNA polymerase, at least one intercalating dye and at least one molecular probe; wherein the kit or the kit of parts is nucleic acid contamination-free; preferably wherein the kit or kit of parts is configured to be operable by or within a cartridge, preferably by or within the cartridge (1a, 1b, 1c) according to the present invention.

One embodiment relates to the kit or the kit of parts according to the present invention, additionally comprising at least one positive control sample.

The at least one positive control sample is a reference sample containing a known quantity of the target DNA sequence, used to verify the proper functioning of the nucleic acid amplification reaction. It ensures that the components of the cartridge (1a, 1b, 1c) is functioning correctly. The positive control must produce the expected amplification product, confirming the validity of the experimental setup and allowing differentiation between true negative results and false negatives due to potential procedural errors.

Preferably, the positive control sample is delivered outside of the cartridge.

A fifth aspect relates to a use of the cartridge (1a, 1b, 1c) according to the present invention, or of the kit or the kit of parts for detecting at least one fungal and/or at least one bacterial contamination in a biological sample with a detection limit of at least 100 CFU, preferably of at least 50 CFU, more preferably of at least 10 CFU.

A Colony Forming Unit (CFU) is a unit used to estimate the number of viable bacteria or fungal cells in a sample. Each CFU represents one or more cells that can multiply to form a colony, which can then be counted. Several methods for applying the CFU measurements are known in the art (e.g., Sieuwerts et al., A simple and fast method for determining colony forming units, Letters in Applied Microbiology (2008), 47, 4, 275-278), Tan et al., Colony-forming unit enumeration by a plate-MPN method, Journal of Food Protection (1983), 46, 10, 836-842).

A sixth aspect relates to a device (7) comprising: (i) the cartridge (1a, 1b, 1c) according to the present invention; (ii) a detection chamber (8) comprising at least one spectral detector, preferably a fluorescence detector; (iii) a control unit (9) programmed to automate the sequential operation of the cartridge (1a, 1b, 1c) as defined in the first aspect and any embodiment thereof; and (iv) optionally: a computer-implemented system (10) for analysing the signals of a qualitative or quantitative nucleic acid amplification reaction.

A spectral detector refers to a device or system designed to detect and measure the intensity of an electromagnetic signal, e.g. fluorescence, emitted by a sample when it is excited by a specific wavelength of light. The spectral detector typically includes components that direct light of a specific wavelength onto the sample, causing the sample to emit fluorescence at a different wavelength. The detector then captures and analyzes the emitted fluorescence, which can be used to identify or quantify specific molecules or compounds within the sample. Quantification is based on an internal control sample include in a storage compartment (5). The key elements of a spectral detector may include a light source, such as a laser or LED, that excites the dye molecules within the sample; optical components, such as lenses, mirrors, and filters, that direct the excitation light to the sample and guide the emitted light to the detection unit; and a detection unit, which may consist of photodetectors, photomultiplier tubes (PMTs), or charge-coupled devices (CCDs), or any other detection unit known in the art that converts the fluorescent light into an electrical signal.

A computer-implemented system is a system that performs specific tasks, processes, or functions using a combination of computer hardware and software. This system typically includes processing units, such as central or graphics processing units that execute software instructions. Memory components, including both volatile and non-volatile storage, are used to store data and programs. The software within the system defines the operations to be carried out, which may involve applications, operating systems, or specialized modules designed for specific purposes.

User interfaces enable interaction with the system, which can be facilitated through graphical or command-line interfaces. Communication interfaces allow the system to connect and interact with other systems, devices, or networks, using various communication protocols.

A seventh aspect relates to a method of testing the nucleic acid contamination of a sample (2), preferably in an automated manner, using the cartridge (1a, 1b, 1c) according to the present invention, or the kit or kit of parts according to the present invention, wherein the method comprises (i) providing a sample to the compartment (2) for receiving the sample; (ii) allowing lysis of a sample in the lysis compartment (3); (iii) allowing binding of the sample of (ii) to the binding material in the nucleic acid purification compartment (4); (iv) performing at least one wash step and at least one elution step in the nucleic acid purification compartment (4) (v) performing a nucleic acid amplification test in the nucleic acid amplification compartment (5); (vi) optionally: obtaining a qualitative or quantitative test result by detection of a spectral signal in the detection chamber (8) according to the present invention; and (vii) optionally: analysing the qualitative or quantitative nucleic acid amplification reaction by the computer implemented system (10) according to the present invention.

### Example 1: DNA depletion using Ethylene Oxide (ETO)

The procedures described in this Example, such as the use of a laminar flow bench for all pipetting activities, UV irradiation of UV-stable materials, and comprehensive cleaning with DNA removers, are specifically designed to eliminate any traces of DNA. These steps are essential in creating an environment that e.g., prevents the introduction of DNA into a cartridge being prepared for sterility testing. Such an environment ensures that the sterility tests conducted are free from the interference of extraneous DNA, which could otherwise compromise the test results.

### Method

To create a DNA-free environment, all pipetting steps were conducted under a laminar flow bench. Before the experiment began, all UV-stable materials, such as pipettes, filter tips, and stands for reaction vessels, were irradiated under the laminar flow bench with UV light for at least half an hour and additionally wiped with DNA Remover^{™} from Minerva Biolabs. All consumables were subjected to ethylene oxide fumigation at 25 °C, 3.5 bar, 120 min, 40% humidity, 15% ethylene oxide (ETO), and 85% CO2. Ethylene oxide easily penetrates cells, where it reacts with molecules containing a labile hydrogen atom to form a hydroxyethyl group. For example, ETO facilitates DNA alkylation, which can lead to DNA strand breaks. One of the main products is N7-hydroxyethylguanine (Setlow et al. Small, Acid-Soluble Spore Proteins of the a/b Type Do Not Protect the DNA in Bacillus subtilis Spores against Base Alkylation, Applied and Environmental Microbiology (1998), 64, 5, 1958-1962).

The potential of ETO treatment for DNA depletion was tested with highly concentrated, lyophilized genomic E. coli DNA. For this, genomic DNA was lyophilized in 1.5 mL reaction vessels, and subsequently, the open vessels were gassed with ETO in a fumigation chamber. The DNA was rehydrated in 10 mM Tris-HCl and analyzed by gel electrophoresis. In parallel, the same sample material was processed identically but without ETO fumigation. As mentioned, DNA alkylation can lead to DNA strand breaks but does not necessarily do so. Nevertheless, alkylated DNA is not amplifiable by DNA polymerase, so the degree of DNA alkylation by ETO is a measure of the efficiency of ETO treatment in depleting DNA. If the ETO-treated DNA were applied directly to an agarose gel, the fragment sizes would only correspond to random strand breaks, not the actual degree of alkylation. An additional piperidine treatment removes all ETO-alkylated bases, causing strand breaks. Therefore, an additional piperidine step shows the actual efficiency of an ETO fumigation and serves not for DNA depletion but only as a confirmation step for the effectiveness of ETO (Setlow et al. 1998).

### Results

The agarose gel electrophoresis depicted in **FIG 5** shows genomic DNA without ETO fumigation in lanes 2 to 4, and ETO-treated DNA in lanes 5 to 8. Lanes 9 to 15 show the same samples as those on the left half of the gel but with additional piperidine treatment. The fragment sizes on the right side of the gel (within the green frame) measure the "natural" or ETO-induced alkylation level of the DNA. It is evident that the fragment sizes in lanes 12 to 15 are significantly smaller than 100 base pairs and that only very weak bands are visible, suggesting a high potential for DNA depletion using ETO. Additionally, the ETO-treated samples were tested in a 16 S rRNA gene based real-time PCR assay. No signal distinguishable from the PCR negative controls was generated. All samples showed no positive signal, indicating that ETO is well-suited for DNA depletion from consumables. ETO is very suitable for dry and solid materials.

### Example 2: Frequency of signals in Negative Extraction Controls (NECs)

This Example demonstrates an evaluation of the Limit of Detection (LOD) using the Microsart^{®} ATMP Bacteria kit and the Microsart^{®} ATMP Sterile Release kit, which are specialized and highly sensitive testing kits for the detection of bacteria and fungi.

### Test procedure

The LOD of multiple species using the Microsart^{®} ATMP Bacteria kit or Microsart^{®} ATMP Sterile Release kit (reagents for Bacteria and Fungi detection) was determined. For every valid DNA extraction and PCR run it is necessary to prove the cleanliness of the experimental process (absence of any contaminating DNA due to the ubiquity of bacterial DNA) by running Negative Extraction Controls (NECs) in parallel to the spiked or unknown samples. Those NECs should not generate any signal in the PCR. This data shows how frequent signals occur in NECs.

### Method

Sample preparation prior testing is strictly required for highest confidence and sensitivity. The design and performance of pre-analytical procedures are part of this study in respect of the intended use but cannot reflect the diversity of the sample material in total. The templates for the PCR analysis are prepared by direct extraction of the sample and subsequent PCR analysis.

### Sample preparation

Microsart^{®} ATMP Extraction and Microsart^{®} ATMP Sterile Release Kit are optimized to extract genomic bacterial and fungal DNA from different sample matrices including cell culture samples, reducing handling steps and thus contamination to a minimum. The Internal Control DNA of Microsart^{®} ATMP Bacteria and Microsart^{®} ATMP Sterile Release can be used to monitor the extraction process. Extraction of bacterial DNA was carried out according to the suitable instruction manual.

In detail:
1. Transfer 1 mL sample into a provided DNA-free 1.5 mL processing tube (transparent cap)
2. Centrifuge at 16200 x g for 15 minutes
3. Remove the supernatant carefully and completely
   Samples can only be inactivated or frozen after this sample collection step.
4. Add 500 µl of Lysis Buffer to the sample
   Optional: Add 20 µl Internal Control DNA from Microsart^{®} ATMP Bacteria kit or from Microsart^{®} ATMP Sterile Release kit to the sample to monitor the extraction process.
5. Vortex vigorously for 30 seconds until pellet is completely lysed
6. Heat at 80°C for 10 minutes
7. Spin down at 16200 x g for 10 minutes
8. Remove supernatant carefully and completely, do not withdraw the pellet
9. Add 100 µl Suspension Buffer (violet cap) and suspend pellet by vortexing

### Analytical procedures

The detection of bacterial DNA will be carried out according to the instruction manual.

### In detail:

### Rehydration of the Reagents:

1. Centrifuge tubes with lyophilized components (5 sec at maximum speed)
2. Add 390 µl (Microsart^{®} ATMP Bacteria) of Rehydration Buffer to each Bacteria Mixes or 90 µl (Microsart^{®} ATMP Sterile Release) of Rehydration Buffer to each Bacteria SR Mixes
3. Add appropriate amount of deionized, DNA-free water
   Positive Control DNA: 300 µl
   Internal Control DNA: 800 µl
4. Incubate for 5 minutes at room temperature
5. Vortex and centrifuge again

### PCR master mix setup:

Total volume per reaction is 25 µl including 10 µl of sample. When setting up reactions, include positive (PC) and negative controls (NTC). Pipet master mix into a 1.5 mL reaction tube and mix gently.

### Pipetting scheme Microsart^{®} ATMP Bacteria (see Table 1)

**Table 1**

| | For 1 reaction | For 26 reactions |
|---|---|---|
| Bacteria mix | 15 µl | 390 µl |
| Internal control DNA | 1 µl | 26 µ |

### Pipetting scheme Microsart^{®} ATMP Sterile Release (see Table 2):

**Table 2**

| Sample | for 1 reaction | 6 reactions |
|---|---|---|
| Bacteria SR Mix | 15 µl | 90.0 µl |
| Internal Control DNA | 1.0 µl | 6.0 µl |

**Attention:** If the Internal Control DNA was added to the sample during DNA extraction, add 15 µl of the Bacteria Mix or Bacteria SR Mix directly to each PCR tube.

### Loading the test tubes:

Aliquot 15 µl of master mix into each PCR reaction tube. After pipetting the negative control (10 µl of water or elution buffer of DNA extraction kit), the tube must be sealed before proceeding with the samples. Add 10 µl of sample to each PCR reaction tube. Seal the tubes completely before proceeding with the positive control (10 µl) in order to avoid cross contamination.

### Programming the real-time PCR cycler:

*Program Step 1: Pre-incubation*
Setting: Hold
Hold Temperature: 95°C
Hold Time: 3 min 0 sec
*Program Step 2: Amplification*
Setting: Cycling
Cycles: 40
Denaturation: 95 °C for 30 sec
Annealing: 55 °C for 30 sec
Detection/ Elongation: 60 °C for 45 sec, FAM^{™} and ROX^{™}

### Result Interpretation

The presence of bacteria in the sample is indicated by an increasing fluorescence signal in the bacterial FAM^{™} channel during PCR. In orderto interpret results accurately, a baseline has to be set. This threshold was defined as 10% of the maximum fluorescence level of the positive control. The presence of bacteria in the sample is indicated by an increasing fluorescence signal in the bacterial FAM^{™} channel during PCR. Internal control (ROX^{™}) must show C_{q}-values in the range of +/- 2 cycles of the no-template control (master mix control) if used as PCR control. If the internal control is used as extraction control it must show C_{q}-values in the range of +/- 3 cycles of the no-template control (master mix control).

To exclude contaminations, the extraction control and the master mix control (FAM^{™}) must be negative (no C_{q}-value or C_{q}-value > 40). The PCR positive control must show C_{q}-values of 24 +/- 2 cycles (FAM^{™}).

**Table 3** shows the system suitability test criteria underlying result interpretation Microsart^{®} ATMP Bacteria and Microsart^{®} ATMP Sterile Release

**Table 3**

| Detection of Bacteria FAM^{™} channel | Internal Control ROX^{™} channel | Interpretation |
|---|---|---|
| positive (C_{q} < 40) | irrelevant | Bacteria positive |
| negative (no C_{q}) | negative (no C_{q}) | PCR inhibition |
| negative (no C_{q}) | positive* | Bacteria negative |

A successfully performed PCR without inhibition is indicated by an increasing fluorescence signal in the internal control channel. The provided Internal Control was added to the PCR master mix or as extraction control. Bacterial DNA and Internal Control DNA are competitors in PCR. Because of the very low concentration of Internal Control in the PCR mix, the signal strength in this channel is reduced with increasing bacterial DNA loads in the sample.

### LOD Detection Limit

As the method employed is used only to obtain a qualitative result, proof of linearity is not required. If however the concept of linearity is extended to cover the working range, the detection limit becomes extremely important. In practice, the detection limit is determined in the form of the positive threshold (i.e., the cut-off point in the form of the minimum number of amplified target sequences by volume positively detected in 95 % of the sample series).

### Procedure

The EZ-CFU of each bacterial species were diluted, according to their individual cell count, in DMEM + 5% FBS to prepare suspensions with concentrations from 99 to 0 CFU/mL. Individual dilution series were prepared for the five bacterial species listed in EP 2.6.1 and EP 2.6.27, available as EZ-CFU, and for another seven bacterial species. Dilutions from 99 to 0 CFU/mL have been tested according to the test procedure described above.

### Acceptance criterion

The cut-off is defined as the lowest bacteria concentration [CFU/mL] which leads in 95% of the tests to a positive result (23/24 and 8/8 samples have to be positive). LOD⁹⁵ must be smaller or equal 99 CFU.

### Results

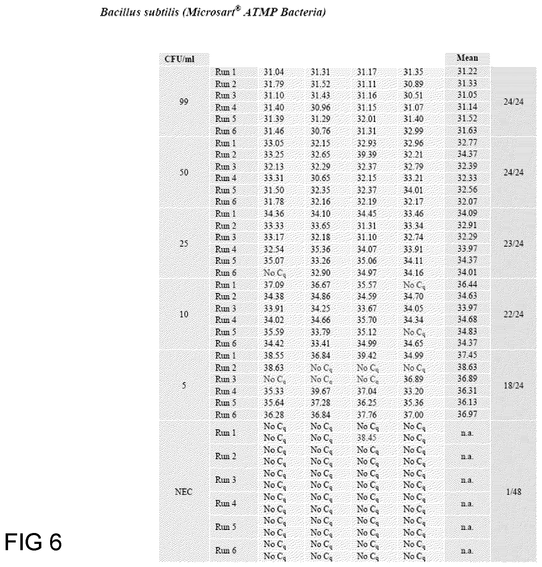

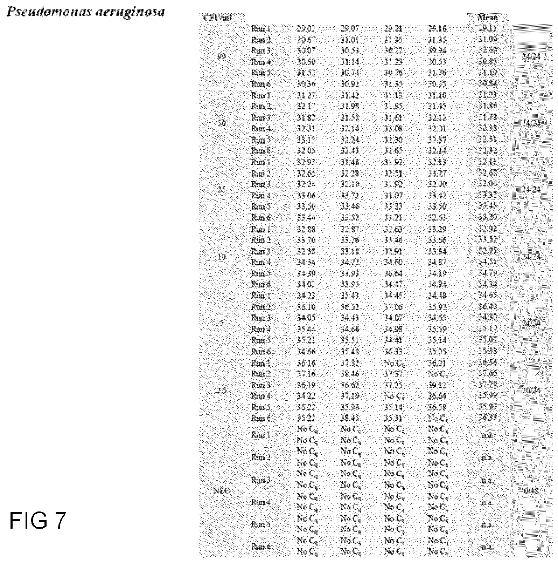

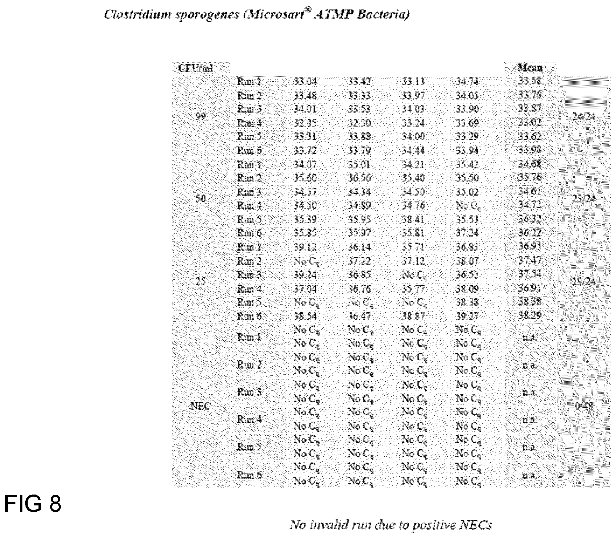

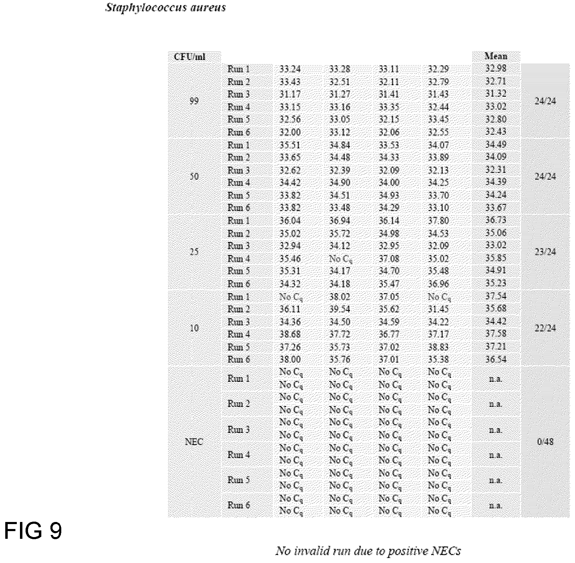

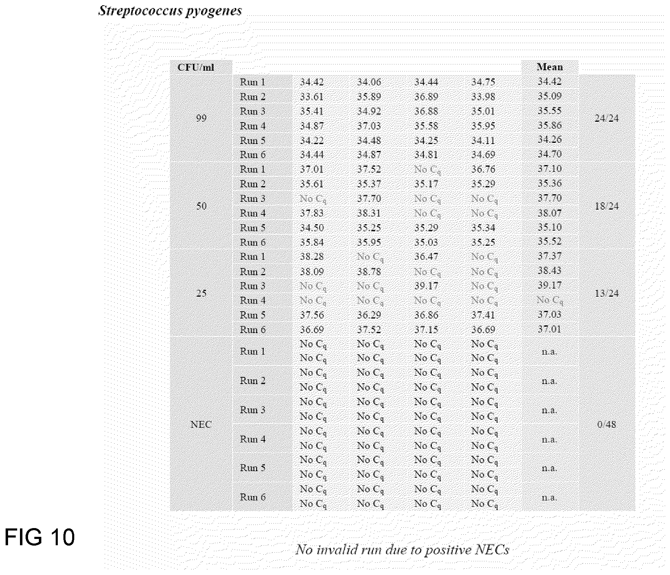

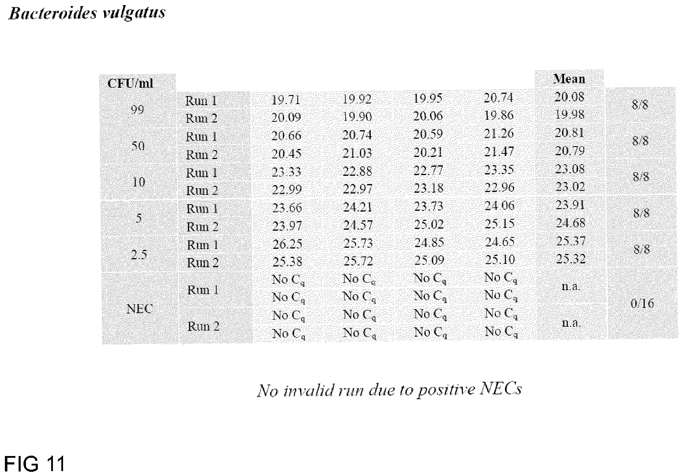

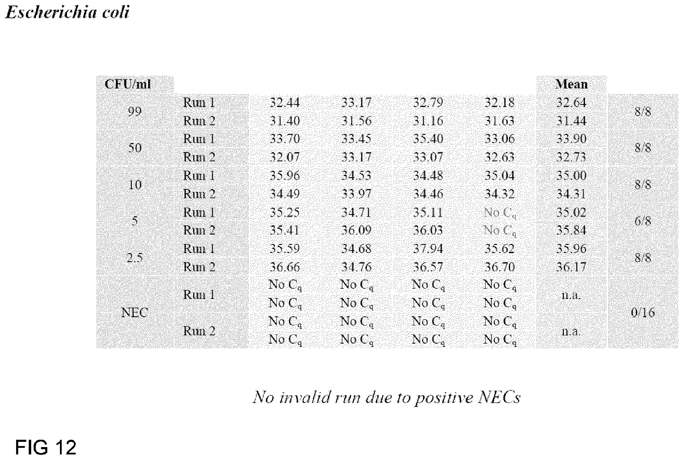

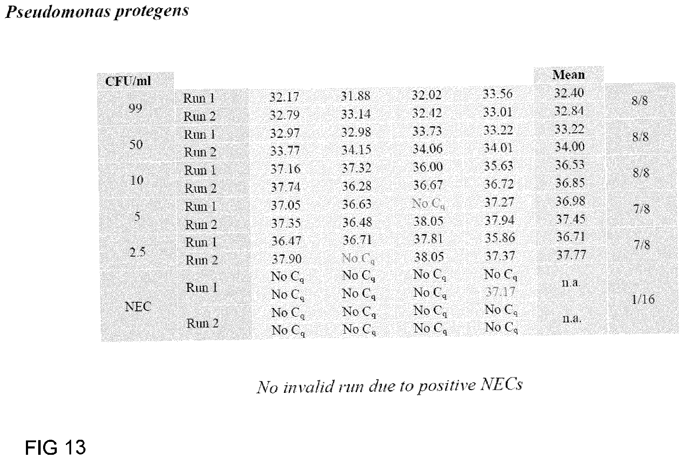

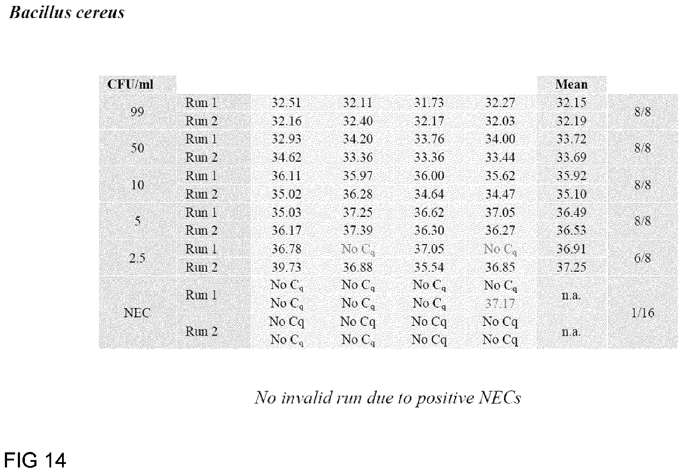

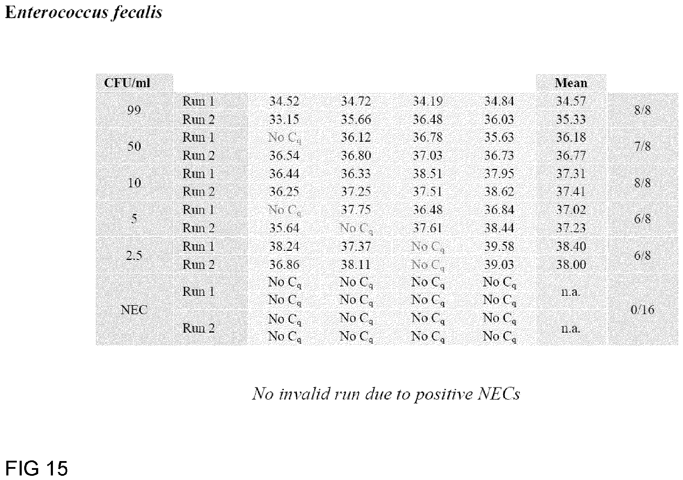

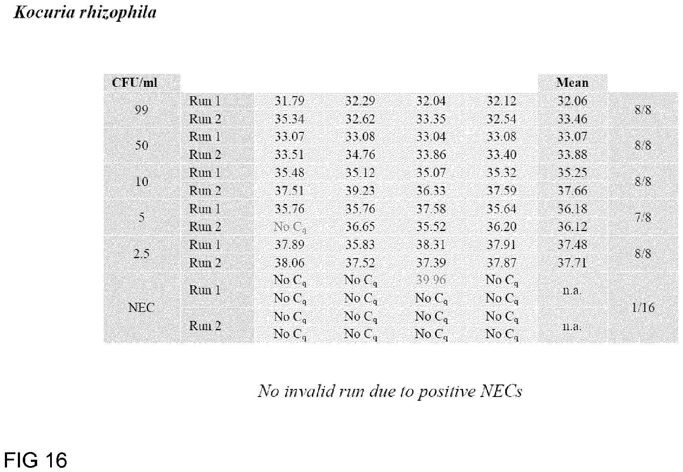

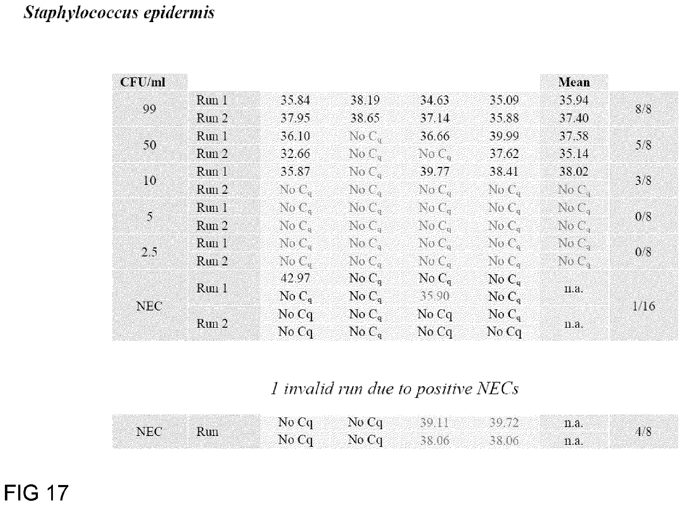

Passed (LOD⁹⁵ comprised between 2.5 CFU and 99 CFU). Tables showing the detailed results for all tested bacterial species are shown in **FIG 6** to **17****.**

### Example 3: DNA depletion methodology

For depletion of residual DNA from reagents, a DNase treatment was performed using RQ1 DNase (Promega). The reaction was carried out with an enzyme concentration of 50 units/mL. Reagents were incubated with the DNase for a minimum of 30 minutes at room temperature. Following incubation, DNase inactivation was achieved by adding 20 mM ethylene glycol tetraacetic acid (EGTA) to the reaction mixture, followed by heating at 60°C for 10 minutes.

Ensuring complete DNase inactivation was critical to avoid degradation of the target DNA during subsequent sample preparation for PCR. Therefore, the effectiveness of the inactivation step was confirmed before using the treated reagents in downstream applications.

### Example 4: Contamination Detection in High Cell Density

### Test procedure

A defined CFU number of the bacterial species *Bacillus subtilis* should be detected in a sample matrix with high cell background (19 x 10⁶ CHO cells per mL) by PCR amplification using the Microsart ATMP Bacteria kit while negative samples remain negative.

### Method

### Preparation of eukaryotic cell background samples

As eukaryotic background CHO cells were used. The CHO cells were diluted with Dulbecco's modified Eagle's medium (DMEM) to the required concentration. For spiked samples, these cell cultures were added to one or more Microsart^{®} Validation Standard to simulate the respective contamination (Positive Validation Standard, PVS). Validation standards of *Bacillus subtilis* were used in the experiment. These standards contain 99 CFU of lyophilized, inactivated bacteria. As a negative control CFU samples with no CFU but only the lyophilization matrix are supplied (Negative Validation Standard, NVS).

### DNA extraction

DNA was extracted with the Sartorius kit Microsart^{®} ATMP Extraction. According to the protocol, 1 mL sample material was added to the DNA free 1.5 mL tubes. The tubes were centrifuged for 15 min at 16 200 g. Supernatant was discarded. To the pellet, 500 µL Lysis Buffer were added. To enable better lysis, the pellet was solved from the bottom of the tube with a pipette tip or by vortexing. After vigorously vortexing for 30 s, the sample was incubated for 10 min at 80 °C and afterwards centrifuged for 10 min at 16.200 g. Supernatant was discarded again. Last, 100 µL Suspension Buffer was added and the tube vortexed vigorously for 30 s. DNA was either used for PCR directly or frozen at - 20 °C.

In experiments without cell background, the first step was the adding of Lysis Buffer in a 1.5 mL tube.

The lysis at 80 °C was also combined with shaking at 1500 rpm, to check the effect of additional shaking during heat lysis.

### Real time polymerase chain reaction (real-time PCR)

For the PCR's Microsart^{®} ATMP Bacteria was used. These Real-time PCR kits use FAM^{™} and ROX^{™} as fluorescence dyes. The dyes are fused to ssDNA probes, which are complementary to the amplified target DNA (FAM^{™}) or the Internal Control (ROX^{™}), which enhances the specificity. FAM^{™} fluorescence signals therefore the targeted amplified DNA, while ROX^{™} fluorescence is used to control the PCR reaction itself in every single reaction.

According to the protocol, the lyophilized Bacteria Mix (MM, master mix), Internal Control and Positive Control were spinned briefly to collect the lyophilizate on the bottom. Then they were rehydrated by adding depending on the kit either 390 µL to the MM and 800 µL PCR grade water to the Internal Control and 300 µL to the Positive Control. The reagents were incubated for 5 min and briefly vortexed afterwards. From the Internal Control, 26 µL were added to the 390 µL MM. Afterwards the PCR was prepared by adding 10 µL sample DNA, PCR grade water (no template control) or Positive Control to 15 µL of the MM in a PCR reaction tube. The complete reaction mix of 25 µL was then amplified according to the following protocol:
1. 95 °C for 3 min
2. 95 °C for 30 s
3. 55 °C for 30 s
4. 60 °C for 45 s

Repeat from 2. 39 x Fluorescence of ROX^{™} and FAM^{™} is measured during the elongation in step 4.

For most experiments all pipetting steps were conducted inside a sterile bench to avoid contaminations and a Stratagene Mx3005p PCR cycler was used. Data were analyzed with the MxPro software. Ct threshold and baseline were manually adjusted for ROX^{™} and FAM^{™} in both software. The baseline was adjusted by setting start and end cycle of the linear phase manually to fitting values. The threshold was set to a tenth of the mean maximum fluorescence of the no template controls (for ROX^{™} data) or the Positive Control (for FAM^{™} data). Samples which result in a Ct value with the set threshold are thereby declared as positive for bacterial content. Samples which do not result in a Ct value are likewise negative.

### Results

Using the described method, the Validation Standard of Bacillus subtilis was successfully detected in High Cell density Background, while no amplification above the threshold line was observed for all negative samples (NVS, NTC). The resulting amplification curves are shown in **FIG 18****.**

### Example 5: List of detectable microorganisms with clinical relevance (see Table 4).

**Table 4**

| **Genus** | **Species** | **Type** of **organism** | **Notes** | **Reference** |
|---|---|---|---|---|
| *Achromobacte r* | *spp.* | bacteria | WFI genus | Sandle, Tim 2015 Characterizing the Microbiota of Pharmaceutical water |
| *Acinetobacter* | *spp.* | bacteria | Listed as common microbial contaminant in biopharm | Clontz 2013 Biopharmaceutical Microbial Contamination Control |
| *Acinetobacter* | *lwoffii* | bacteria | Identified by Penna, BMC Public health 2002:2:13 | Sandle, Tim 2015 Characterizing the Microbiota of Pharmaceutical water |
| *Acinetobacter* | *spp.* | bacteria | WFI genus | Sandle, Tim 2015 Characterizing the Microbiota of Pharmaceutical water |
| *Acinetobacter* | *johnsonii* | bacteria | Main genera from pyrosequencing reads | Park 2014 Bacterial Diversity in the indoor air of pharmaceutical environment |
| *Acinetobacter* | *junii* | bacteria | Main genera from pyrosequencing reads | Park 2014 Bacterial Diversity in the indoor air of pharmaceutical environment |
| *Acremonium* | | fungi | Black Magic Tatoo ink | Vijayakumar, R & Sandle 2012 Review of fungal contaminants in pharmaceuticals |
| *Actinomycetes* | *spp.* | bacteria | Listed as common microbial contaminant in biopharm | Clontz 2013 Biopharmaceutical Microbial Contamination Control |
| *Aeromonas* | *spp.* | bacteria | WFI genus | Sandle, Tim 2015 Characterizing the Microbiota of Pharmaceutical water |
| *Alcaligenes* | *spp.* | bacteria | WFI genus | Sandle, Tim 2015 Characterizing the Microbiota of Pharmaceutical water |
| *Alternaria* | *spp.* | fungi | Review of melanized fungal contamination | Vijayakumar, R. 2016 A Review of Melanized fungal contaminants |
| *Alternaria* | *alternata* | fungi | J&J containmated Risperdal | Vijayakumar, R. 2016 A Review of Melanized fungal contaminants |
| *Alternaria* | *spp.* | fungi | Listed as common microbial contaminant in biopharm | Clontz 2013 Biopharmaceutical Microbial Contamination Control |
| *Arthrobacter* | *spp.* | bacteria | Listed as common microbial contaminant in biopharm | Clontz 2013 Biopharmaceutical Microbial Contamination Control |
| Aspergillus | spp. | fungi | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Aspergillus* | *spp.* | fungi | Review of melanized fungal contamination | Vijayakumar, R. 2016 A Review of Melanized fungal contaminants |
| *Aspergillus* | *fumigatu* s | fungi | Lubriderm Mitts | Vijayakumar, R & Sandle 2012 Review of fungal contaminants in pharmaceuticals |
| *Aspergillus* | *versicolo r* | fungi | Lubriderm Mitts | Vijayakumar, R & Sandle 2012 Review of fungal contaminants in pharmaceuticals |
| *Aspergillus* | *niger* | fungi | | Vijayakumar, R & Sandle 2012 Review of fungal contaminants in pharmaceuticals |
| *Aspergillus* | *spp.* | fungi | Most common isolates | Rodriguez 2013 MS Thesis - Microbial survey of Ajinomoto Althea Environmental Isolates |
| *Aspergillus* | *spp.* | fungi | Listed as common microbial contaminant in biopharm | Clontz 2013 Biopharmaceutical Microbial Contamination Control |
| *Aspergillus brasiliensis Aspergillus fumigatus IHEM 22670* | | *Mold* | | USP<71>/Ph. Eur. 2.6.1/JP 54/ Ph. Eur. 2.6.27 *from Cytotherapy 2014* |
| *Aspergillus niger (ATCC 16404)* | | | | *from Cytotherapy 2004* |
| Bacillus | spp. | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Bacillus* | *sphaeric uslfusifo rmis* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Bacillus* | *pumilis* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Bacillus* | *cereus* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Bacillus* | *cereus* | bacteria | Fermentor for protein product secreted by bacterial cells | Vijayakumar, R & Sandle 2012 Review of fungal contaminants in pharmaceuticals |
| *Bacillus* | *spp.* | bacteria | Mains water system | Sandle, Tim 2015 Characterizing the Microbiota of Pharmaceutical water |
| *Bacillus* | *cereus* | bacteria | Identified microbes from cold rooms | Sandle, Tim 2013 Psychrophilic and psychrotolerant microbes in cold rooms |
| *Bacillus* | *sphaeric us*/*fusifo rmis* | bacteria | Identified microbes from cold rooms | Sandle, Tim 2013 Psychrophilic and psychrotolerant microbes in cold rooms |
| *Bacillus* | *spp.* | bacteria | Most common genus - all human microbes | Rodriguez 2013 MS Thesis - Microbial survey of Ajinomoto Althea Environmental Isolates |
| *Bacillus* | *cereus* | bacteria | Most common isolates | Rodriguez 2013 MS Thesis - Microbial survey of Ajinomoto Althea Environmental Isolates |
| *Bacillus* | *spp.* | bacteria | Main genera from pyrosequencing reads | Park 2014 Bacterial Diversity in the indoor air of pharmaceutical environment |
| *Bacillus* | *spp.* | bacteria | Listed as common microbial contaminant in biopharm | Clontz 2013 Biopharmaceutical Microbial Contamination Control |
| *Bacillus cereus ATCC* 14579 | | | | *from Cytotherapy 2014* |
| *Bacillus spp.* | | | | *from Cytotherapy 2014* |
| *Bacillus subtilis* | | *Bacteria, Gram positive rod* | | USP<71>/Ph. Eur. 2.6.1/JP 54/ Ph. Eur. 2.6.27 |
| *Bacillus subtilis (ATCC* 6633) | | | | *from Cytotherapy 2004* |
| *Bacteroides fragilis* ATCC 25285 | | | | *from HPC sterility testing* |
| *Bacteroides spp.* | | | | *from Cytotherapy 2014* |
| *Bifidobacteriu m spp.* | | | | *from Cytotherapy 2014* |
| *Biopolaris* | *hawaiien sis* | fungi | 40 patients via a compounding lab | Vijayakumar, R. 2016 A Review of Melanized fungal contaminants |
| *Bordetella* | *spp.* | bacteria | WFI genus | Sandle, Tim 2015 Characterizing the Microbiota of Pharmaceutical water |
| *Brevundimona* s | *spp.* | bacteria | Mains water system | Sandle, Tim 2015 Characterizing the Microbiota of Pharmaceutical water |
| *Brevundimona* s | *vesicular is* | bacteria | Mains water system | Sandle, Tim 2015 Characterizing the Microbiota of Pharmaceutical water |
| *Brevundimona* s | *spp.* | bacteria | WFI genus | Sandle, Tim 2015 Characterizing the Microbiota of Pharmaceutical water |
| *Brevundimona* s | *vesicular is* | bacteria | WFI species | Sandle, Tim 2015 Characterizing the Microbiota of Pharmaceutical water |
| *Brevundimona s vesicularis CRB-FC 54.27* | | | | *from Cytotherapy 2014* |
| *Burkholderia* | *cepacia* | bacteria | Medline Baby lotions & oils | Vijayakumar, R & Sandle 2012 Review of fungal contaminants in pharmaceuticals |
| *Burkholderia* | *spp.* | bacteria | Mains water system | Sandle, Tim 2015 Characterizing the Microbiota of Pharmaceutical water |
| *Burkholderia* | *spp.* | bacteria | genera from purified water systems | Sandle, Tim 2015 Characterizing the Microbiota of Pharmaceutical water |
| *Burkholderia* | *cepacia* | bacteria | spp from purified water systems | Sandle, Tim 2015 Characterizing the Microbiota of Pharmaceutical water |
| *Burkholderia* | *spp.* | bacteria | WFI genus | Sandle, Tim 2015 Characterizing the Microbiota of Pharmaceutical water |
| *Burkholderia* | *cepacia* | bacteria | WFI species | Sandle, Tim 2015 Characterizing the Microbiota of Pharmaceutical water |
| *Burkholderia* | *cepacia* | bacteria | Identified microbes from cold rooms | Sandle, Tim 2013 Psychrophilic and psychrotolerant microbes in cold rooms |
| *Burkholderia* | *spp.* | bacteria | Listed as common microbial contaminant in biopharm | Clontz 2013 Biopharmaceutical Microbial Contamination Control |
| *C albicans* | | | | *from Cytotherapy 2004* |
| *C sporogenes* | | | | *from Cytotherapy 2004* |
| *Candida* | *lipolytica* | fungi | Identified microbes from cold rooms | Sandle, Tim 2013 Psychrophilic and psychrotolerant microbes in cold rooms |
| *Candida* | spp. | fungi | Listed as common microbial contaminant in biopharm | Clontz 2013 Biopharmaceutical Microbial Contamination Control |
| *Candida albicans Candida albicans (ATCC 10231)* | | *Yeast* | | USP<71>/Ph. Eur. 2.6.1/JP 54/ Ph. Eur. 2.6.27 *from Cytotherapy 2014* |
| *Candida albicans ATCC 90028* | | | | *from Cytotherapy 2014* |
| *Candida glabrata IHEM 19160* | | | | *from Cytotherapy 2014* |
| *Candida krusei ATCC* 6228 | | | | *from Cytotherapy 2014* |
| *Candida parapsilosis ATCC 22019* | | | | *from Cytotherapy 2014* |
| *Chryseobacter ium* | *spp.* | bacteria | WFI genus | Sandle, Tim 2015 Characterizing the Microbiota of Pharmaceutical water |
| *Chryseobacter ium* | *indologe nes* | bacteria | WFI species | Sandle, Tim 2015 Characterizing the Microbiota of Pharmaceutical water |
| *Chryseobacter ium* | *indologe nes* | bacteria | Identified microbes from cold rooms | Sandle, Tim 2013 Psychrophilic and psychrotolerant microbes in cold rooms |
| *Chryseobacter ium* | *spp.* | bacteria | Main genera from pyrosequencing reads | Park 2014 Bacterial Diversity in the indoor air of pharmaceutical environment |
| *Chryseobacter ium meningoseptic* um | | | | *from Cytotherapy 2014* |
| *Chryseomona* s | *spp.* | bacteria | WFI genus | Sandle, Tim 2015 Characterizing the Microbiota of Pharmaceutical water |
| *Cladosporium* | *spp.* | fungi | Review of melanized fungal contamination | Vijayakumar, R. 2016 A Review of Melanized fungal contaminants |
| *Cladosporium* | *spp.* | fungi | IV bags of antibiotics at Claris Life Sciences | Vijayakumar, R. 2016 A Review of Melanized fungal contaminants |
| *Cladosporium* | *spp.* | fungi | Listed as common microbial contaminant in biopharm | Clontz 2013 Biopharmaceutical Microbial Contamination Control |
| *Cladosporium sphaerospermum* 18883 | *IHEM* | | | *from Cytotherapy 2014* |
| *Cloacibacteriu m* | *normane nse* | bacteria | Main genera from pyrosequencing reads | Park 2014 Bacterial Diversity in the indoor air of pharmaceutical environment |
| *Clostridium* | *spp.* | bacteria | Listed as common microbial contaminant in biopharm | Clontz 2013 Biopharmaceutical Microbial Contamination Control |
| *Clostridium perfringens* ATCC 13 124 | | | | *from HPC sterility testing* |
| *Clostridium sporogenes* | | *Anaerobic bacteria, Gram positive rod* | | USP<71>/Ph. Eur. 2.6.1/JP 54/ Ph. Eur. 2.6.27 |
| *Clostridium spp.* | | | | *from Cytotherapy 2014* |
| *Coagulase-negative Staphylococcu* s | | | | *from Cytotherapy 2014* |
| *Comamonas* | *spp.* | bacteria | WFI genus | Sandle, Tim 2015 Characterizing the Microbiota of Pharmaceutical water |
| Corynebacteri um | spp. | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Corynebacteri um* | *spp*. | bacteria | Most common genus - all human microbes | Rodriguez 2013 MS Thesis - Microbial survey of Ajinomoto Althea Environmental Isolates |
| *Corynebacteri um* | *spp*. | bacteria | Main genera from pyrosequencing reads | Park 2014 Bacterial Diversity in the indoor air of pharmaceutical environment |
| *Corynebacteri um* | *spp*. | bacteria | Listed as common microbial contaminant in biopharm | Clontz 2013 Biopharmaceutical Microbial Contamination Control |
| *Corynebacterium amycolatum ATCC 700207* | | | | *from Cytotherapy 2014* |
| *Corynebacteri um spp*. | | | | *from Cytotherapy 2014* |
| *Cryptococcus* | *spp.* | fungi | Listed as common microbial contaminant in biopharm | Clontz 2013 Biopharmaceutical Microbial Contamination Control |
| *Cupriavidus* | *spp.* | bacteria | WFI genus | Sandle, Tim 2015 Characterizing the Microbiota of Pharmaceutical water |
| *Curvularia* | *spp.* | fungi | Review of melanized fungal contamination | Vijayakumar, R. 2016 A Review of Melanized fungal contaminants |
| *Diaphorobacte r* | *nitroredu cens* | bacteria | Main genera from pyrosequencing reads | Park 2014 Bacterial Diversity in the indoor air of pharmaceutical environment |
| *Enterobacteria ceae* | | | | *from Cytotherapy 2014* |
| *Enterococcus spp.* | | | | *from Cytotherapy 2014* |
| *Epidermophyt on* | *spp.* | fungi | Review of melanized fungal contamination | Vijayakumar, R. 2016 A Review of Melanized fungal contaminants |
| *Escherichia* | *coli* | bacteria | Most common isolates | Rodriguez 2013 MS Thesis - Microbial survey of Ajinomoto Althea Environmental Isolates |
| *Escherichia coli ATCC* 25922 | | | | *from Cytotherapy 2014* |
| *Eubacterium spp.* | | | | *from Cytotherapy 2014* |
| *Exophiala* | | fungi | Introduced in a compounding pharmacy, 2002 | Vijayakumar, R. 2016 A Review of Melanized fungal contaminants |
| *Exserohilum* | *rostratu m* | fungi | 750 patients via compounding lab | Vijayakumar, R. 2016 A Review of Melanized fungal contaminants |
| *Flavimonas* | *spp.* | bacteria | genera from purified water systems | Sandle, Tim 2015 Characterizing the Microbiota of Pharmaceutical water |
| *Flavimonas* | *oryzihab itans* | bacteria | spp from purified water systems | Sandle, Tim 2015 Characterizing the Microbiota of Pharmaceutical water |
| *Flavimonas* | *spp.* | bacteria | WFI genus | Sandle, Tim 2015 Characterizing the Microbiota of Pharmaceutical water |
| *Flavimonas* | *oryzihab itans* | bacteria | WFI species | Sandle, Tim 2015 Characterizing the Microbiota of Pharmaceutical water |
| *Flavobacteriu m* | *aureum* | bacteria | Identified by Penna, BMC Public health 2002:2:13 | Sandle, Tim 2015 Characterizing the Microbiota of Pharmaceutical water |
| *Flavobacteriu m* | *multivor um* | bacteria | Identified by Penna, BMC Public health 2002:2:13 | Sandle, Tim 2015 Characterizing the Microbiota of Pharmaceutical water |
| *Flavobacteriu m* | *spp.* | bacteria | Main genera from pyrosequencing reads | Park 2014 Bacterial Diversity in the indoor air of pharmaceutical environment |
| *Fluviicola* | taffensis | bacteria | Main genera from pyrosequencing reads | Park 2014 Bacterial Diversity in the indoor air of pharmaceutical environment |
| *Fusarium* | *spp*. | fungi | Review of melanized fungal contamination | Vijayakumar, R. 2016 A Review of Melanized fungal contaminants |
| *Fusarium* | *spp*. | fungi | B&L Contact lens soln | Vijayakumar, R & Sandle 2012 Review of fungal contaminants in pharmaceuticals |
| *Fusarium* | *spp*. | fungi | Listed as common microbial contaminant in biopharm | Clontz 2013 Biopharmaceutical Microbial Contamination Control |
| *Fusobacterium* naviforme ATCC 25832 | | | | *from HPC sterility testing* |
| *Gordonia* | *spp*. | bacteria | WFI genus | Sandle, Tim 2015 Characterizing the Microbiota of Pharmaceutical water |
| *Kitasatospora* | *spp*. | bacteria | Main genera from pyrosequencing reads | Park 2014 Bacterial Diversity in the indoor air of pharmaceutical environment |
| *Klebsiella* | *spp*. | bacteria | Listed as common microbial contaminant in biopharm | Clontz 2013 Biopharmaceutical Microbial Contamination Control |
| *Klebsiella pneumoniae (ATCC 700603)* | | | | *from Cytotherapy 2004* |
| *Lactobacillus* species (Lab isolate) | | | | *from HPC sterility testing* |
| *Lactobacillus spp.* | | | | *from Cytotherapy 2014* |
| *Lichtheimia corymbifera* | | | formerly Absidia corymbifera IHEM 16288 | *from Cytotherapy 2014* |
| *Limnohabitans* | *plankton icuslpar vus* | bacteria | Main genera from pyrosequencing reads | Park 2014 Bacterial Diversity in the indoor air of pharmaceutical environment |
| *Methylobacteri um* | *spp.* | bacteria | Mains water system | Sandle, Tim 2015 Characterizing the Microbiota of Pharmaceutical water |
| *Methylobacteri um* | *spp.* | bacteria | WFI genus | Sandle, Tim 2015 Characterizing the Microbiota of Pharmaceutical water |
| *Methylobacteri um* | *spp.* | bacteria | Most common isolates | Rodriguez 2013 MS Thesis - Microbial survey of Ajinomoto Althea Environmental Isolates |
| *Microbacteriu m* | *spp.* | bacteria | WFI genus | Sandle, Tim 2015 Characterizing the Microbiota of Pharmaceutical water |
| *Microbacteriu m* | *spp.* | bacteria | Identified microbes from cold rooms | Sandle, Tim 2013 Psychrophilic and psychrotolerant microbes in cold rooms |
| *Micrococcus* | spp. | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Micrococcus* | *luteus* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Micrococcus* | *lylae* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Micrococcus* | *spp.* | bacteria | Mains water system | Sandle, Tim 2015 Characterizing the Microbiota of Pharmaceutical water |
| *Micrococcus* | *luteus* | bacteria | Identified microbes from cold rooms | Sandle, Tim 2013 Psychrophilic and psychrotolerant microbes in cold rooms |
| *Micrococcus* | *spp.* | bacteria | Most common genus - all human microbes | Rodriguez 2013 MS Thesis - Microbial survey of Ajinomoto Althea Environmental Isolates |
| *Micrococcus* | *kocuria* | bacteria | Most common isolates | Rodriguez 2013 MS Thesis - Microbial survey of Ajinomoto Althea Environmental Isolates |
| *Micrococcus* | *spp.* | bacteria | Listed as common microbial contaminant in biopharm | Clontz 2013 Biopharmaceutical Microbial Contamination Control |
| *Micrococcus luteus (ATCC 9341*) | | | | *from Cytotherapy 2004* |
| *Microsporon* | *spp.* | fungi | Review of melanized fungal contamination | Vijayakumar, R. 2016 A Review of Melanized fungal contaminants |
| *Moraxella* | *spp.* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Moraxella* | *spp.* | bacteria | Mains water system | Sandle, Tim 2015 Characterizing the Microbiota of Pharmaceutical water |
| *Moraxella* | *spp.* | bacteria | genera from purified water systems | Sandle, Tim 2015 Characterizing the Microbiota of Pharmaceutical water |
| *Moraxella* | *spp.* | bacteria | WFI genus | Sandle, Tim 2015 Characterizing the Microbiota of Pharmaceutical water |
| *Moraxella* | *spp.* | bacteria | WFI species | Sandle, Tim 2015 Characterizing the Microbiota of Pharmaceutical water |
| *Mycobacteriu m* | *spp.* | bacteria | Main genera from pyrosequencing reads | Park 2014 Bacterial Diversity in the indoor air of pharmaceutical environment |
| *myroides* | *spp.* | bacteria | WFI genus | Sandle, Tim 2015 Characterizing the Microbiota of Pharmaceutical water |
| *Ochrobactrum* | *anthropi* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Ochrobactrum* | *spp.* | bacteria | genera from purified water systems | Sandle, Tim 2015 Characterizing the Microbiota of Pharmaceutical water |
| *Ochrobactrum* | *anthropi* | bacteria | spp from purified water systems | Sandle, Tim 2015 Characterizing the Microbiota of Pharmaceutical water |
| *Ochrobactrum* | *spp.* | bacteria | WFI genus | Sandle, Tim 2015 Characterizing the Microbiota of Pharmaceutical water |
| *P acnes* | | | | *from Cytotherapy 2004* |
| *P aeruginosa* | | | | *from Cytotherapy 2004* |
| *Paenibacillus* | *curdlano lyticus* | bacteria | fermentor used to manufacture recombinant protein | Vijayakumar, R & Sandle 2012 Review of fungal contaminants in pharmaceuticals |
| *Paenibacillus* | *spp.* | bacteria | Most common isolates | Rodriguez 2013 MS Thesis - Microbial survey of Ajinomoto Althea Environmental Isolates |
| *Pantoea* | *spp.* | bacteria | WFI genus | Sandle, Tim 2015 Characterizing the Microbiota of Pharmaceutical water |
| Penicillin | spp. | fungi | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Penicillium* | *spp*. | fungi | Review of melanized fungal contamination | Vijayakumar, R. 2016 A Review of Melanized fungal contaminants |
| *Penicillium* | *spp*. | fungi | Lubriderm Mitts | Vijayakumar, R & Sandle 2012 Review of fungal contaminants in pharmaceuticals |
| *Penicillium* | *spp*. | fungi | Most common isolates | Rodriguez 2013 MS Thesis - Microbial survey of Ajinomoto Althea Environmental Isolates |
| *Penicillium* | *spp*. | fungi | Listed as common microbial contaminant in biopharm | Clontz 2013 Biopharmaceutical Microbial Contamination Control |
| *Penicillium chrysogenum IHEM 22667* | | | | *from Cytotherapy 2014* |
| *Peptostreptoc occus spp.* | | | | *from Cytotherapy 2014* |
| *Pityosporum* | ovale | fungi | Review of melanized fungal contamination | Vijayakumar, R. 2016 A Review of Melanized fungal contaminants |
| *Pityosporum* | orbicular e | fungi | Review of melanized fungal contamination | Vijayakumar, R. 2016 A Review of Melanized fungal contaminants |
| *Porphyromona s spp.* | | | | *from Cytotherapy 2014* |
| *Prevotella spp.* | | | | *from Cytotherapy 2014* |
| *Propionibacter ium* | *acnes* | bacteria | Main genera from pyrosequencing reads | Park 2014 Bacterial Diversity in the indoor air of pharmaceutical environment |
| *Propionibacter ium acnes* | | *Anaerobic bacteria, Gram positive rod* | | Ph. Eur. 2.6.27 |
| *Propionibacter ium acnes (ATCC 11827)* | | | | *from Cytotherapy 2014* |
| *Propionibacter ium acnes (ATCC 6919)* | | | | *from Cytotherapy 2014* |
| *Propionibacter* ium acnes (Lab isolate) | | | | *from HPC sterility testing* |
| *Propionibacter ium spp.* | | | | *from Cytotherapy 2014* |
| *Pseudomonas* | *oryzihab itans* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Pseudomonas* | *aerugino sa* | bacteria | Black Magic Tatoo ink | Vijayakumar, R & Sandle 2012 Review of fungal contaminants in pharmaceuticals |
| *Pseudomonas* | *aerugino sa* | bacteria | Identified by Penna, BMC Public health 2002:2:13 | Sandle, Tim 2015 Characterizing the Microbiota of Pharmaceutical water |
| *Pseudomonas* | *vesicular is* | bacteria | Identified by Penna, BMC Public health 2002:2:13 | Sandle, Tim 2015 Characterizing the Microbiota of Pharmaceutical water |
| *Pseudomonas* | *diminuta* | bacteria | Identified by Penna, BMC Public health 2002:2:13 | Sandle, Tim 2015 Characterizing the Microbiota of Pharmaceutical water |
| *Pseudomonas* | *flouresc ens* | bacteria | Identified by Penna, BMC Public health 2002:2:13 | Sandle, Tim 2015 Characterizing the Microbiota of Pharmaceutical water |
| *Pseudomonas* | *putida* | bacteria | Identified by Penna, BMC Public health 2002:2:13 | Sandle, Tim 2015 Characterizing the Microbiota of Pharmaceutical water |
| *Pseudomonas* | *alcaligen es* | bacteria | Identified by Penna, BMC Public health 2002:2:13 | Sandle, Tim 2015 Characterizing the Microbiota of Pharmaceutical water |
| *Pseudomonas* | *paucimo bills* | bacteria | Identified by Penna, BMC Public health 2002:2:13 | Sandle, Tim 2015 Characterizing the Microbiota of Pharmaceutical water |
| *Pseudomonas* | *spp.* | bacteria | Mains water system | Sandle, Tim 2015 Characterizing the Microbiota of Pharmaceutical water |
| *Pseudomonas* | *stutzeri* | bacteria | Mains water system | Sandle, Tim 2015 Characterizing the Microbiota of Pharmaceutical water |
| *Pseudomonas* | *spp.* | bacteria | genera from purified water systems | Sandle, Tim 2015 Characterizing the Microbiota of Pharmaceutical water |
| *Pseudomonas* | *spp.* | bacteria | WFI genus | Sandle, Tim 2015 Characterizing the Microbiota of Pharmaceutical water |
| *Pseudomonas* | *flouresc ens* | bacteria | WFI species | Sandle, Tim 2015 Characterizing the Microbiota of Pharmaceutical water |
| *Pseudomonas* | *flouresc ens* | bacteria | Identified microbes from cold rooms | Sandle, Tim 2013 Psychrophilic and psychrotolerant microbes in cold rooms |
| *Pseudomonas* | *spp.* | bacteria | Most common isolates | Rodriguez 2013 MS Thesis - Microbial survey of Ajinomoto Althea Environmental Isolates |
| *Pseudomonas* | *spp.* | bacteria | Main genera from pyrosequencing reads | Park 2014 Bacterial Diversity in the indoor air of pharmaceutical environment |
| *Pseudomonas* | *spp.* | bacteria | Listed as common microbial contaminant in biopharm | Clontz 2013 Biopharmaceutical Microbial Contamination Control |
| *Pseudomonas aeruginosa* | | *Bacteria, Gram negative rod* | | USP<71>/Ph. Eur. 2.6.1/JP 54/ Ph. Eur. 2.6.27 |
| *Pseudomonas aeruginosa (ATCC 27853)* | | | | *from Cytotherapy 2014* |
| *Pseudomonas aeruginosa (ATCC 9027)* | | | | *from Cytotherapy 2014* |
| *Pseudomonas fluorescens ATCC 13525* | | | | *from Cytotherapy 2014* |
| *Ralstonia* | *pickettii* | bacteria | detected in the UF/DF step in prelicense inspections, likely a biofilm | Vijayakumar, R & Sandle 2012 Review of fungal contaminants in pharmaceuticals |
| *Ralstonia* | *pickettii* | bacteria | Identified by Penna, BMC Public health 2002:2:13 | Sandle, Tim 2015 Characterizing the Microbiota of Pharmaceutical water |
| *Ralstonia* | *spp.* | bacteria | Mains water system | Sandle, Tim 2015 Characterizing the Microbiota of Pharmaceutical water |
| *Ralstonia* | *spp.* | bacteria | genera from purified water systems | Sandle, Tim 2015 Characterizing the Microbiota of Pharmaceutical water |
| *Ralstonia* | *pickettii* | bacteria | spp from purified water systems | Sandle, Tim 2015 Characterizing the Microbiota of Pharmaceutical water |
| *Ralstonia* | *spp.* | bacteria | WFI genus | Sandle, Tim 2015 Characterizing the Microbiota of Pharmaceutical water |
| *Ralstonia* | *pickettii* | bacteria | WFI species | Sandle, Tim 2015 Characterizing the Microbiota of Pharmaceutical water |
| *Ralstonia* | *spp.* | bacteria | Listed as common microbial contaminant in biopharm | Clontz 2013 Biopharmaceutical Microbial Contamination Control |
| *Rhizopus* | *microsp orus* | fungi | Allopurinol | Vijayakumar, R & Sandle 2012 Review of fungal contaminants in pharmaceuticals |
| *Rhodococcus* | *spp.* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Rhodococcus* | *spp.* | bacteria | Identified microbes from cold rooms | Sandle, Tim 2013 Psychrophilic and psychrotolerant microbes in cold rooms |
| *Rhodotorula S aureus* | *spp.* | fungi | Listed as common microbial contaminant in biopharm | Clontz 2013 Biopharmaceutical Microbial Contamination Control *from Cytotherapy 2004* |
| *Serratia* | *spp.* | bacteria | WFI genus | Sandle, Tim 2015 Characterizing the Microbiota of Pharmaceutical water |
| *Serratia* | *spp.* | bacteria | Listed as common microbial contaminant in biopharm | Clontz 2013 Biopharmaceutical Microbial Contamination Control |
| *Sphingomona s* | *spp.* | bacteria | detected in the UF/DF step in prelicense inspections, likely a biofilm | Vijayakumar, R & Sandle 2012 Review of fungal contaminants in pharmaceuticals |
| *Sphingomona s* | *spp.* | bacteria | Mains water system | Sandle, Tim 2015 Characterizing the Microbiota of Pharmaceutical water |
| *Sphingomona s* | *spp.* | bacteria | WFI genus | Sandle, Tim 2015 Characterizing the Microbiota of Pharmaceutical water |
| *Sphingomona s koreensis* | | | | *from Cytotherapy 2014* |
| *CRB-FC* 229.82 | | | | |
| Staphylococcu s | spp. | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Staphylococcu* s | *epidermi dis* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Staphylococcu* s | *capitis* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Staphylococcu* s | *hominis* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Staphylococcu* s | *haemoly ficus* | bacteria | Review of 9000 microbial isolates from a range of different grade cleanrooms. Most are human isolates, 2001-2009 | Sandle, Tim. A Review of Cleanroom Microflora: Types, Treds and Patterns. Journal of Pharmaceutical Science and Technology. Vol 65, No. 4, July-Aug 2011 |
| *Staphylococcu* s | *spp.* | bacteria | detected in the UF/DF step in prelicense inspections, likely a biofilm | Vijayakumar, R & Sandle 2012 Review of fungal contaminants in pharmaceuticals |
| *Staphylococcu* s | *spp.* | bacteria | Most common genus - all human microbes | Rodriguez 2013 MS Thesis - Microbial survey of Ajinomoto Althea Environmental Isolates |
| *Staphylococcu* s | *epidermi dis* | bacteria | Most common isolates | Rodriguez 2013 MS Thesis - Microbial survey of Ajinomoto Althea Environmental Isolates |
| *Staphylococcu* s | *aureus* | bacteria | Most common isolates | Rodriguez 2013 MS Thesis - Microbial survey of Ajinomoto Althea Environmental Isolates |
| *Staphylococcu* s | *epidermi dis* | bacteria | Main genera from pyrosequencing reads | Park 2014 Bacterial Diversity in the indoor air of pharmaceutical environment |
| *Staphylococcu* s | *Saproph yticus* | bacteria | Main genera from pyrosequencing reads | Park 2014 Bacterial Diversity in the indoor air of pharmaceutical environment |
| *Staphylococcu* s | *spp.* | bacteria | Listed as common microbial contaminant in biopharm | Clontz 2013 Biopharmaceutical Microbial Contamination Control |
| *Staphylococcu* s *aureus* | | *Bacteria, Gram positive cocci* | | USP<71>/Ph. Eur. 2.6.1/JP 54/ Ph. Eur. 2.6.27 |
| *Staphylococcu* s *aureus (ATCC 6538 or 6583)* | | | | *from Cytotherapy 2014* |
| *Staphylococcu s epidermidis CIP 68.21* | | | | *from Cytotherapy 2014* |
| *Stenotrophom onas* | *maltophi lia* | bacteria | detected in the UF/DF step in prelicense inspections, likely a biofilm | Vijayakumar, R & Sandle 2012 Review of fungal contaminants in pharmaceuticals |
| *Stenotrophom onas* | *spp.* | bacteria | Mains water system | Sandle, Tim 2015 Characterizing the Microbiota of Pharmaceutical water |
| *Stenotrophom onas* | *spp.* | bacteria | genera from purified water systems | Sandle, Tim 2015 Characterizing the Microbiota of Pharmaceutical water |
| *Stenotrophom onas* | *maltophi lia* | bacteria | spp from purified water systems | Sandle, Tim 2015 Characterizing the Microbiota of Pharmaceutical water |
| *Stenotrophom onas* | *spp.* | bacteria | WFI genus | Sandle, Tim 2015 Characterizing the Microbiota of Pharmaceutical water |
| *Stenotrophom onas* | *maltophi lia* | bacteria | WFI species | Sandle, Tim 2015 Characterizing the Microbiota of Pharmaceutical water |
| *Stenotrophom onas* | *spp.* | bacteria | Listed as common microbial contaminant in biopharm | Clontz 2013 Biopharmaceutical Microbial Contamination Control |
| *Streptococcus* | *spp.* | bacteria | Listed as common microbial contaminant in biopharm | Clontz 2013 Biopharmaceutical Microbial Contamination Control |
| *Streptococcus pyogenes* | | *Bacteria, Gram positive cocci* | | Ph. Eur. 2.6.27 |
| *Streptococcus spp.* | | | | *from Cytotherapy 2014* |
| *Streptomyces* | *spp.* | bacteria | Main genera from pyrosequencing reads | Park 2014 Bacterial Diversity in the indoor air of pharmaceutical environment |
| *Trichomacaea* | *spp.* | fungi | Most common isolates | Rodriguez 2013 MS Thesis - Microbial survey of Ajinomoto Althea Environmental Isolates |
| *Trichophyton* | *spp.* | fungi | Review of melanized fungal contamination | Vijayakumar, R. 2016 A Review of Melanized fungal contaminants |
| *Wautersia* | *spp.* | bacteria | WFI genus | Sandle, Tim 2015 Characterizing the Microbiota of Pharmaceutical water |
| *Yersinia* | *intermed ia* | bacteria | Identified microbes from cold rooms | Sandle, Tim 2013 Psychrophilic and psychrotolerant microbes in cold rooms |
| *Yersinia enterocolitica* | | *Bacteria, Gram negative rod* | | Ph. Eur. 2.6.27 |
| *Yersinia enterocolitica (ATCC 9610)* | | | | *from Cytotherapy 2004* |

## Claims

1. A cartridge (1a, 1b, 1c) for performing a sequence of steps of a nucleic acid amplification test on a sample, comprising:
(i) at least one compartment (2) for receiving the sample;
(ii) at least one lysis compartment (3);
(iii) at least one nucleic acid purification compartment (4);
(iv) at least one compartment (5) comprising a set of nucleic acid amplification reagents, and
(v) optionally: at least one waste compartment (6);
wherein the cartridge (1a, 1b, 1c), the compartments and all reagents are provided nucleic acid contamination-free;
wherein the cartridge (1a, 1b, 1c) is configured to be operable by a device allowing an automated execution of sample preparation and a nucleic acid amplification test, preferably obtaining a qualitative or quantitative test result;
wherein the cartridge (1a, 1b, 1c) allows nucleic acid contamination-free fluid transfer between different, or all, compartments comprised;
optionally: wherein two or more compartments are physically identical.

2. The cartridge (1a, 1b, 1c) according to claim 1, wherein the cartridge, the compartments and all reagents are nucleic acid contamination-free as determined by having a Ct value of at least 35, preferably a Ct value of at least 40 for a respective assay target.

3. The cartridge (1a, 1b, 1c) according to claims 1 and 2, wherein the sample is
(i) a pharmaceutical sample; or
(ii) a biological specimen with a final cellular concentration of at least 0.5 x 10⁶ cells per mL, more preferably of at least 1 x 10⁶ cells per mL, most preferably of at least 10 x 10⁶ cells per mL.

4. The cartridge (1a, 1b, 1c) according to any one of the preceding claims, wherein the lysis compartment (3) comprises at least one chaotropic agent, preferably guanidine hydrochloride, in a final concentration range of 0.1 to 8 M, preferably 0.2 to 7 M, more preferably 0.5 to 6 M, preferably at least one protease with a final concentration range of 10 to 2,000 µg/mL, preferably 20 to 1,500 µg/mL, more preferably 50 to 1,000 µg/mL, and/or optionally at least one RNase in a final concentration range of 10 to 500 µg/mL, preferably 20 to 300 µg/mL, more preferably 50 to 200 µg/mL, and/or optionally comprises a mechanical disruptor and/or at least one disintegrant supporting lysis of the sample.

5. The cartridge (1a, 1b, 1c) according to any one of the preceding claims, wherein the nucleic acid purification compartment (4) comprises a nucleic acid binding material with capacity to process nucleic acids corresponding to at least 100 bacterial / fungi CFU within the nucleic acid background corresponding to at least 0.5 x 10⁶ eukaryotic cells, more preferably at least 1 x 10⁶ eukaryotic cells, most preferably at least 10 x 10⁶ eukaryotic cells, optionally, at least one reconstitution buffer, at least one binding buffer, at least one wash buffer and at least one elution buffer.

6. The cartridge (1a, 1b, 1c) according to any one of the preceding claims, wherein the set of nucleic acid amplification reagents comprises at least one nucleic acid amplification buffer, optionally an RT polymerase, at least one DNA polymerase, a set of primers, an internal control sample, and at least one intercalating dye and/or at least one molecular probe.

7. The cartridge (1a, 1b, 1c) according to any one of the preceding claims, wherein nucleic acid amplification test detects at least 90%, preferably at least 94% of all known bacterial species and/or at least 30%, preferably at least 37%, of all known fungal species, including all clinically relevant bacterial and/or fungal species.

8. The cartridge (1a, 1b, 1c) according to any one of the preceding claims, wherein the at least one compartment (5) additionally comprises a microfluidic device configured to partition a nucleic acid sample into discrete entities suitable for digital nucleic acid amplification and, optionally suitable for obtaining a qualitative or quantitative test result.

9. A use of the cartridge (1a, 1b, 1c) as defined in any one of the preceding claims for performing a nucleic acid amplification test on a sample as defined in claim 3.

10. A method of producing the cartridge (1a, 1b, 1c) as defined in claims 1 to 8, wherein assembly of individual components is performed in a nucleic acid-free environment, preferably with DNA depleted components, comprising the following steps:
(i) optionally: DNA-depleting all individual and/or pre-assembled components and/or reagents of the cartridge (1a, 1b, 1c);
(ii) assembling the housing of the cartridge (1a, 1b, 1c);
(iii) inserting into the housing of the cartridge (1a, 1b, 1c),
(a) at least one compartment (2) for receiving the sample;
(b) at least one lysis compartment (3);
(c) at least one nucleic acid purification compartment (4);
(d) at least one compartment (5), optionally comprising a set of nucleic acid amplification reagents, and
(e) optionally: at least one waste compartment (6);
(iv) sealing the cartridge (1a, 1b, 1c), preferably in a nucleic acid contamination-free environment.

11. A kit or a kit of parts comprising at least one nucleic acid amplification reagent selected from the group consisting of at least one lysis buffer, at least one binding material, at least one binding buffer, at least one wash buffer, at least one elution buffer, at least one nucleic acid amplification buffer, at least one nucleic acid primer, at least one internal control, optionally at least one RT polymerase, at least one DNA polymerase, at least one intercalating dye and at least one molecular probe;
wherein the kit or the kit of parts is nucleic acid contamination-free;
preferably wherein the kit or kit of parts is configured to be operable by or within a cartridge, preferably by or within the cartridge (1a, 1b, 1c) as defined in claims 1 to 8.

12. The kit or the kit of parts according to claim 11 additionally comprising at least one positive control sample.

13. A use of the cartridge (1a, 1b, 1c) according to claims 1 to 8, or of the kit or the kit of parts for detecting at least one fungal and/or at least one bacterial contamination in a biological sample with a detection limit of at least 100 CFU, preferably of at least 50 CFU, more preferably of at least 10 CFU.

14. A device (7) comprising:
(i) the cartridge (1a, 1b, 1c) according to claims 1 to 8;
(ii) a detection chamber (8) comprising at least one spectral detector, preferably a fluorescence detector;
(iii) a control unit (9) programmed to automate the sequential operation of the cartridge (1a, 1b, 1c) as defined in claims 1 to 8; and
(iv) optionally: a computer-implemented system (10) for analysing the signals of a qualitative or quantitative nucleic acid amplification reaction.

15. A method of testing the nucleic acid contamination of a sample (2), preferably in an automated manner, using the cartridge (1a, 1b, 1c) according to claims 1 to 8, or the kit or kit of parts according to claims 11 and 12, wherein the method comprises
(i) providing a sample to the compartment (2) for receiving the sample;
(ii) allowing lysis of a sample in the lysis compartment (3);
(iii) allowing binding of the sample of (ii) to the binding material in the nucleic acid purification compartment (4);
(iv) performing at least one wash step and at least one elution step in the nucleic acid purification compartment (4);
(v) performing a nucleic acid amplification test in the nucleic acid amplification compartment (5);
(vi) optionally: obtaining a qualitative or quantitative test result by detection of a spectral signal in the detection chamber (8) as defined in claim 14; and
(vii) optionally: analysing the qualitative or quantitative nucleic acid amplification reaction by the computer implemented system (10) as defined in claim 14.
